# EUROPÄISCHE PATENTSCHRIFT

(11) **EP 3 774 761 B1**
(45) Veröffentlichungstag und Bekanntmachung des Hinweises auf die Patenterteilung: **11.09.2024**
(21) Anmeldenummer: 19715090.7
(22) Anmeldetag: 03.04.2019
(51) Int. Cl.: C07D 317/72, C07C 229/48, C07D 491/10, C07D 498/10, C07C 233/52

(54) **VERFAHREN ZUR HERSTELLUNG VON SUBSTITUIERTEN CYCLOHEXANAMINOSÄUREESTERN UND SPIROKETALSUBSTITUIERTEN CYCLISCHEN KETOENOLEN**
METHOD FOR THE PREPARATION OF CYCLOHEXANAMINO ACID ESTERS AND SPIROKETAL SUBSTITUTED CYCLIC KETOENOLS
PROCÉDÉ DE PRODUCTION D'ESTERS D'ACIDE AMINÉ SUBSTITUÉS PAR CYCLOHEXANE ET DE KÉTOÉNOLES CYCLIQUES SUBSTITUÉS PAR SPIROKÉTAL

(30) Priorität: 10.04.2018 EP 18166442
(43) Veröffentlichungstag der Anmeldung: 17.02.2021
(73) Patentinhaber: Bayer Aktiengesellschaft, 51373 Leverkusen (DE)
(72) Erfinder: HIMMLER, Thomas, 51519 Odenthal (DE); BRÜCHNER, Peter, 47809 Krefeld (DE); LINDNER, Werner, 51067 Köln (DE); HAHN, Julia, Johanna, 40589 Düsseldorf (DE); MORADI, Wahed, Ahmed, 40789 Monheim (DE); FISCHER, Reiner, 40789 Monheim (DE); DOCKNER, Michael, 50674 Köln (DE)
(74) Vertreter: BIP Patents
(86) Internationale Anmeldenummer: PCT/EP2019/058354
(87) Internationale Veröffentlichungsnummer: WO 2019/197231

(56) Entgegenhaltungen:
- EP-A2- 3 301 092

## Beschreibung

Die vorliegende Erfindung betrifft ein neues Verfahren zur Herstellung von substituierten Cyclohexanaminosäureestern und spiroketal-substituierten cyclischen Ketoenolen sowie neue Intermediate bzw. Ausgangsverbindungen, welche in dem erfindungsgemäßen Verfahren durchlaufen bzw. verwendet werden. Substituierte cyclische Cyclohexanaminosäureester sind wichtige Zwischenprodukte zur Synthese von insektiziden, akariziden und herbiziden Wirkstoffen.

Es ist bereits bekannt geworden, dass bestimmte spiroketal-substituierte cyclische Ketoenole insektizide, akarizide oder herbizide Wirksamkeit zeigen (WO 99/16748, WO 06/089633). Eine bekannt gewordene Synthese (A) (WO 2014/024659) solcher spiroketal-substituierter cyclischer Ketoenole geht von entsprechend spiroketal-substituierten Cyclohexanonen der allgemeinen Formel (I) aus, die in einer Bucherer-Bergs-Reaktion zu den spiroketal-substituierten Hydantoinen der allgemeinen Formel (II) umgesetzt werden. Alkalische Verseifung dieser Hydantoine ergibt die spiroketal-substituierten Cyclohexanaminosäuren der allgemeinen Formel (III). Diese Aminosäuren werden dann durch Umsetzung mit Methanol und Thionylchlorid zu einem Gemisch der Hydrochloride der substituierten Cyclohexanaminosäuremethylester der allgemeinen Formeln (IV), (V) und (VI) verestert. Aus diesen Hydrochloriden können mittels Basen die freien Cyclohexanaminosäuremethylester der allgemeinen Formeln (IV), (V) und (VI) erhalten werden,. Diese Aminosäuremethylester werden dann mit Phenylessigsäurechloriden der allgemeinen Formel (VII) am Stickstoff zu einem Gemisch der Verbindungen der allgemeinen Formeln (VIII), (IX) und (X) acyliert. Die Verbindungen der allgemeinen Formeln (VIII), (IX) und (X) werden dann in einer Dieckmann-Reaktion durch Einwirkung einer starken Base wie Kalium-tertiärbutylat oder Natriummethanolat zu einem Gemisch der substituierten cyclischen Ketoenole der allgemeinen Formeln (XI), (XII) und (XIII) cyclisiert. In einem letzten Schritt werden diese Verbindungen dann durch Reaktion mit einem a,ω̅-Diol der allgemeinen Formel (XIV) in die Verbindung der allgemeinen Formel (XI) umgewandelt. Dieses Verfahren (A) ist in Schema 1 abgebildet. Ein erheblicher Nachteil dieses Verfahrens (A) besteht darin, dass bei der Veresterung der Aminosäuren der allgemeinen Formel (III) mit Methanol/Thionylchlorid es auch zur Bildung von Methylchlorid kommt. Methylchlorid entweicht wegen seines niedrigen Siedepunktes (-24°C) mit dem Abgas. Seine Entsorgung (beispielsweise durch Verbrennung) kann ein gravierendes technisches Problem darstellen.

EP 3301092 A2 beschreibt ein Verfahren zur Herstellung von spiroketal-substituierten Phenylacetylaminosäureestern und spiroketal-substituierten cyclischen Ketoenole, wobei keine n-propylsubstituierten Verbindungen (R⁷ = n-Propyl) beschrieben werden.

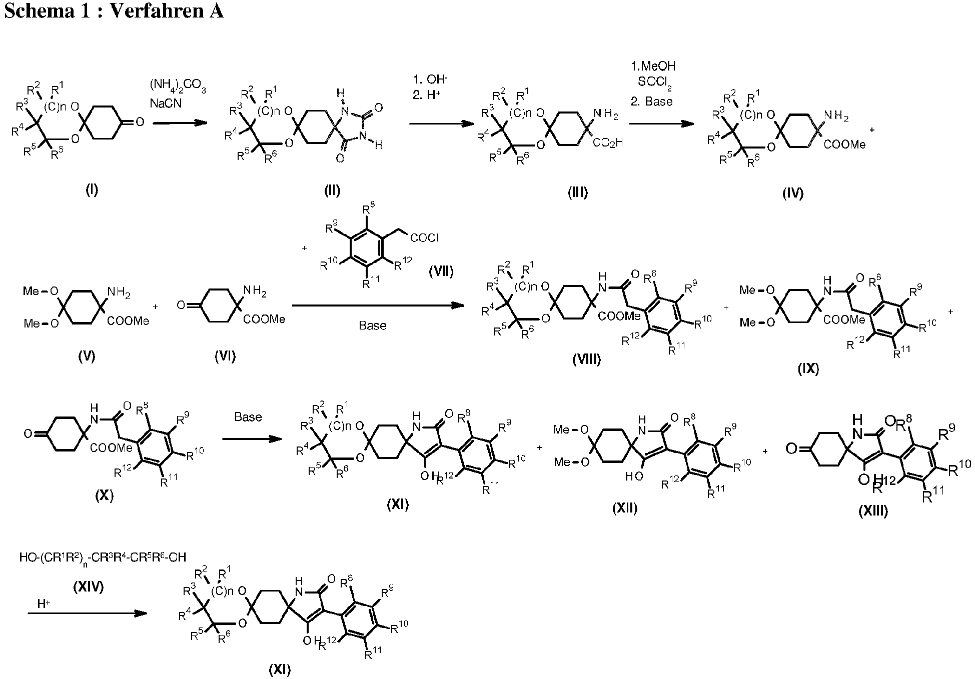

In den allgemeinen Formeln (I), (II), (III), (IV), (V), (VI), (VII), (VIII), (IX), (X), (XI), (XII), (XIII) und (XIV) haben R¹, R², R³, R⁴, R⁵, R⁶, R⁸, R⁹, R¹⁰, R¹¹, R¹² und n die unten angegebenen Bedeutungen.

Es bestand daher weiterhin Bedarf an einem unter technischen Bedingungen besser durchführbaren Verfahren zur Herstellung von spiroketal-substituierten Cyclohexanaminosäureestern der allgemeinen Formel (VIII) und spiroketal-substituierten cyclischen Ketoenolen der allgemeinen Formel (XI).

Es wurde nun gefunden, dass sich die Synthese von spiroketal-substituierten Cyclohexanaminosäureestern der allgemeinen Formel (VIII) und von spiroketal-substituierten cyclischen Ketoenolen der allgemeinen Formel (XI) vereinfachen lässt, indem man anstelle von Methanol höhere Alkohole verwendet, deren entsprechende Alkylchloride infolge ihrer höheren Siedepunkte leichter nach prinzipiell bekannten technischen Methoden aus dem Abgasstrom entfernt werden können bzw. nicht mehr in diesem auftreten. Außerdem wurde gefunden, dass überraschenderweise bei der Reaktion mit höheren Alkoholen als Methanol die Bildung der Dialkyl-acetale deutlich reduziert ist. Dies vereinfacht die Reaktionssequenz und verkürzt den letzten Schritt des Verfahrens.

Eine erste Ausführungsform (Verfahren B) des erfindungsgemäßen Verfahrens ist dadurch gekennzeichnet, dass man spiroketal-substituierte Cyclohexanaminosäuren der allgemeinen Formel (III) durch Umsetzung mit einem Alkohol der allgemeinen Formel (XV)

R⁷-OH (XV),

in der
- R⁷: für n-Propyl steht,
und Thionylchlorid zu Gemischen der Hydrochloride der spiroketal-substituierten Cyclohexanaminosäureester der allgemeinen Formel (IV'), dialkylketal-substituierten Cyclohexanaminosäureester der allgemeinen Formel (V') und 4-Cyclohexanon-aminosäureestern der allgemeinen Formel (VI') verestert. Aus diesen Hydrochloriden können mittels Basen die freien spiroketal-substituierten Cyclohexanaminosäureester der allgemeinen Formel (IV'), dialkylketal-substituierten Cyclohexanaminosäureester der allgemeinen Formel (V`) und 4-Cyclohexanon-aminosäureestern der allgemeinen Formel (VI') erhalten werden. Diese Aminosäureester werden dann in Gegenwart einer Base mit Phenylessigsäurechloriden der allgemeinen Formel (VII) am Stickstoff acyliert, wobei Gemische der Verbindungen der allgemeinen Formeln (VIII`), (IX') und (X') erhalten werden. Diese Verbindungen werden anschließend in einer Dieckmann-Reaktion durch Einwirkung einer starken Base wie Kalium-tertiärbutylat oder Natriummethanolat zu Gemischen der Verbindungen der allgemeinen Formeln (XI), (XII') und (XIII) cyclisiert. Abschließend werden diese Verbindungen dann durch Umsetzung in Gegenwart einer Säure mit einem α,ω̅-Diol der allgemeinen Formel (XIV)

HO-(CR' R²)ₙ-CR³R⁴-CR⁵R⁶-OH (XIV),

in welcher
- R³: für Wasserstoff steht,
- R⁴: für Wasserstoff steht,
- R⁵: für Wasserstoff steht,
- R⁶: für Wasserstoff steht,
und
- n: für 0 steht,
da n für 0 steht, sind die Substituenten R¹ und R² nicht definiert,
in ein einheitliches spiroketal-substituiertes Ketoenol der allgemeinen Formel (XI)
umgewandelt.

Das erfindungsgemäße Verfahren (B) ist in Schema 2 abgebildet.

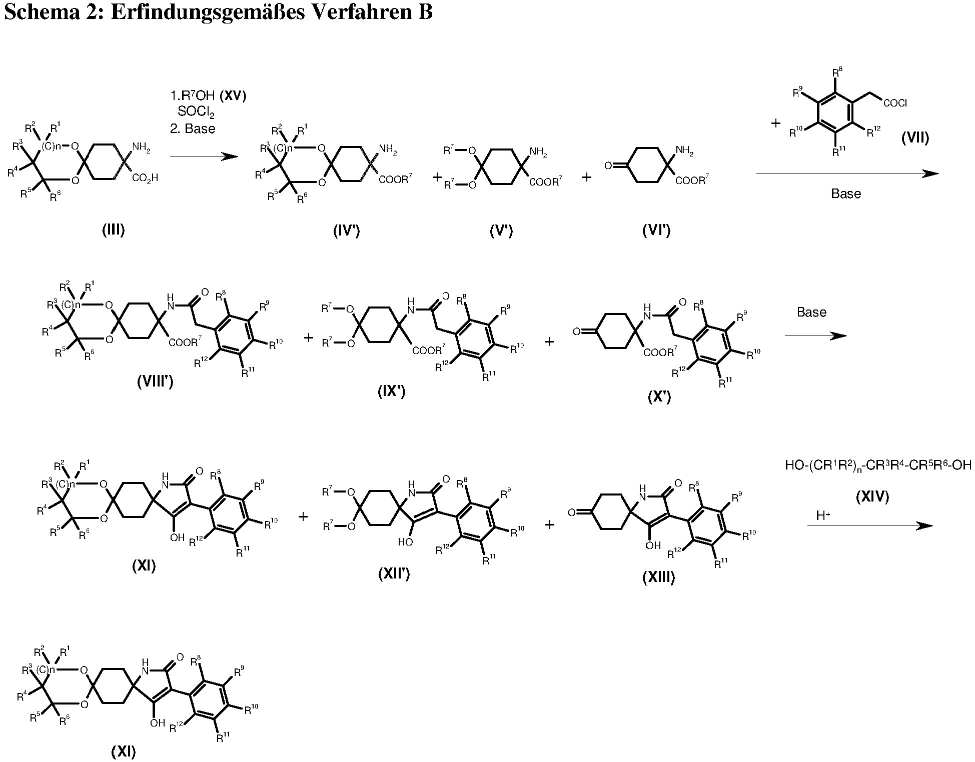

Eine zweite Ausführungsform (Verfahren C) des erfindungsgemäßen Verfahrens ist dadurch gekennzeichnet, dass man spiroketal-substituierte Cyclohexanaminosäuren der allgemeinen Formel (III) durch Umsetzung mit einem Alkohol der allgemeinen Formel (XV)

R⁷-OH (XV),

in der
- R⁷: für n-Propyl steht,
und Thionylchlorid zu Gemischen der Hydrochloride der spiroketal-substituierten Cyclohexanaminosäureester der allgemeinen Formel (IV'), dialkylketal-substituierten Cyclohexanaminosäureester der allgemeinen Formel (V') und 4-Cyclohexanon-aminosäureestern der allgemeinen Formel (VI') verestert. Aus diesen Hydrochloriden können mittels Basen die freien spiroketal-substituierten Cyclohexanaminosäureester der allgemeinen Formel (IV'), dialkylketal-substituierten Cyclohexanaminosäureester der allgemeinen Formel (V`) und 4-Cyclohexanon-aminosäureestern der allgemeinen Formel (VI') erhalten werden. Diese Aminosäureester werden dann in Gegenwart einer Base mit Phenylessigsäurechloriden der allgemeinen Formel (VII) am Stickstoff acyliert, wobei Gemische der Verbindungen der allgemeinen Formeln (VIII`), (IX') und (X') erhalten werden.

Diese Verbindungen werden dann durch Umsetzung in Gegenwart einer Säure mit einem (α,ω̅-Diol der allgemeinen Formel (XIV)

HO-(CR¹R²)ₙ-CR³R⁴-CR⁵R⁶-OH (XIV),

in welcher
- R³: für Wasserstoff steht,
- R⁴: für Wasserstoff steht,
- R⁵: für Wasserstoff steht,
- R⁶: für Wasserstoff steht,
und
- n: für 0 steht,
da n für 0 steht, sind die Substituenten R¹ und R² nicht definiert,
in die einheitlich spiroketal-substituierte Verbindung der allgemeinen Formel (XVI) 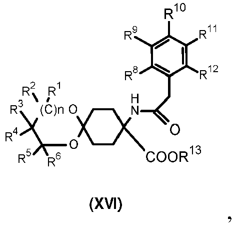 in welcher
- R¹³: für n-Propyl oder -CH₂CH₂-OH
steht,
überführt. Abschließend wird dann die Verbindung der Formel (XVI) in einer Dieckmann-Reaktion durch Einwirkung einer starken Base (wie beispielsweise Kalium-tertiärbutylat oder Natriummethanolat) zu der Verbindung der Formel (XI) cyclisiert.

Das erfindungsgemäße Verfahren (C) ist in Schema 3 abgebildet.

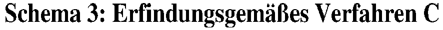

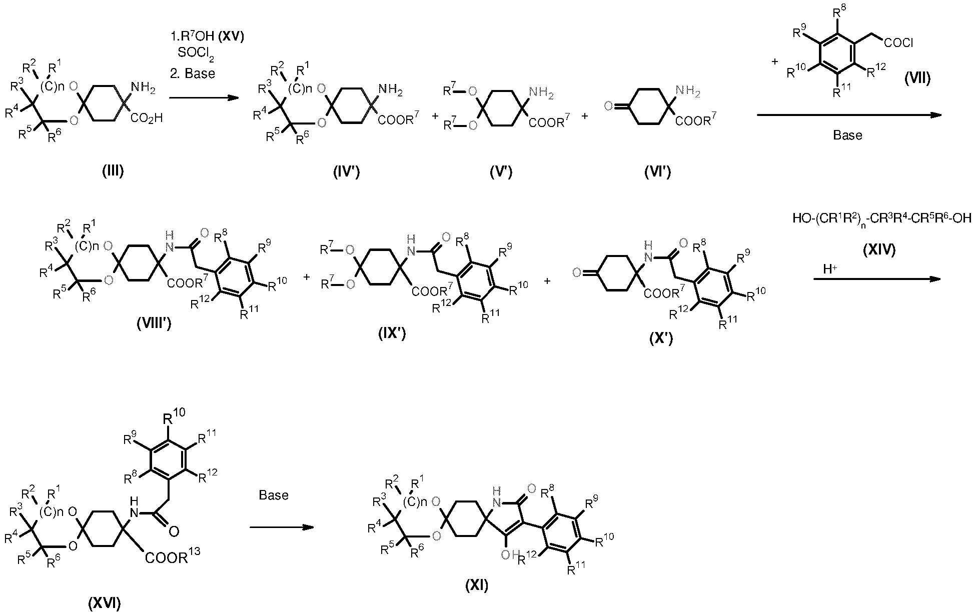

In den allgemeinen Formeln (III), (IV`), (V'), (VI'), (VII), (VIII`), (IX'), (X'), (XI), (XII'), (XIII), (XIV), (XV) und (XVI) stehen
- R³: für Wasserstoff,
- R⁴: für Wasserstoff,
- R⁵: für Wasserstoff,
- R⁶: für Wasserstoff,
- R⁷: für n-Propyl,
- R⁸: für Methyl,
- R⁹: für Wasserstoff,
- R¹⁰: für Chlor,
- R¹¹: für Wasserstoff,
- R¹²: für Methyl,
- R¹³: für n-Propyl oder -CH₂CH₂-OH,
- n: für 0.

Im Folgenden wird das erfindungsgemäße Verfahren (B) näher erläutert.

Erster Schritt (1) des erfindungsgemäßen Verfahrens (B): Die Reaktion von Verbindungen der allgemeinen Formel (III) mit einem Alkohol der allgemeinen Formel (XV) und Thionylchlorid zu den Hydrochloriden der Verbindungen der allgemeinen Formeln (IV`), (V`) und (VI') kann ohne Verdünnungsmittel oder gegebenenfalls in Gegenwart eines inerten Verdünnungsmittel wie beispielsweise Toluol, Chlorbenzol, 1,2-Dichlorbenzol, Heptan, Isooctan, Methylcyclohexan, Anisol oder Acetonitril durchgeführt werden. Bevorzugt arbeitet man ohne Verdünnungsmittel, d.h. der zur Veresterung verwendete Alkohol wird im Überschuß als Verdünnungsmittel eingesetzt.

Die Menge an Thionylchlorid kann in weiten Grenzen variiert werden. Üblicherweise arbeitet man mit 0,5 bis 5 Moläquivalenten Thionylchlorid, bezogen auf Verbindung der allgemeinen Formel (III). Bevorzugt verwendet man 0,9 bis 3 Moläquivalente Thionylchlorid. Besonders bevorzugt verwendet man 1,2 bis 3 Moläquivalente Thionylchlorid.

Die Reaktionstemperatur liegt zwischen -10 und 150°C, bevorzugt zwischen 0 und 120°C.

Die Reaktion kann grundsätzlich auch bei vermindertem oder erhöhtem Druck durchgeführt werden.

Die Aufarbeitung kann beispielsweise durch Abdestillieren des Alkohols und überschüssigen Thionylchlorids erfolgen. Auf diese Weise erhält man die Hydrochloride der Verbindungen der allgemeinen Formeln (IV`), (V`) und (VI`), die als solche in den nächsten Schritt des erfindungsgemäßen Verfahrens (B) eingesetzt werden können.

Es ist jedoch auch möglich, die Hydrochloride der Verbindungen der allgemeinen Formeln (IV'), (V') und (VI') durch Zusatz einer Base in die freien Aminoesterverbindungen der allgemeinen Formeln (IV`), (V`) und (VI') zu überführen und diese durch übliche Aufarbeitungsmethoden wie Filtration oder Extraktion zu isolieren.

Als Base können inerte anorganische und organische Basen verwendet werden wie beispielsweise Natriumcarbonat, Natriumhydrogencarbonat, Kaliumcarbonat, Kaliumhydrogencarbonat, Lithiumhydroxid, Natriumhydroxid, Kaliumhydroxid, Ammoniak, Triethylamin, Tributylamin, Pyridin oder 2-Methyl-5-ethyl-pyridin oder Gemische dieser Basen.. Bevorzugt wird Natriumcarbonat verwendet. Ebenfalls bevorzugt verwendet man das Gemisch von Natriumcarbonat mit Natriumhydroxid.

Die Menge an Base wird so gewählt, dass der Anteil an Hydrochlorid (HCl) im Gemisch der Hydrochloride der Verbindungen der allgemeinen Formeln (IV`), (V`) und (VI') neutralisiert wird.

Zweiter Schritt (2) des erfindungsgemäßen Verfahrens (B): Die Hydrochloride der Verbindungen der allgemeinen Formeln (IV'), (V') und (VI') oder die Verbindungen der allgemeinen Formeln (IV'), (V') und (VI') werden in Gegenwart eines inerten Verdünnungsmittels und einer Base mit einem Säurechlorid der allgemeinen Formel (VII) zu Verbindungen der allgemeinen Formeln (VIII'), (IX') und (X') umgesetzt.

Als Verdünnungsmittel können beispielsweise Dichlormethan, Toluol, Xylol, Chlorbenzol, 1,2-Dichlorbenzol, Heptan, Isooctan, Methylcyclohexan, Tetrahydrofuran, Essigester, Acetonitril, Anisol oder Butyronitril verwendet werden. Bevorzugt verwendet man Tetrahydrofuran (THF), Anisol, Toluol, Xylol, Chlorbenzol oder Acetonitril. Besonders bevorzugt verwendet man Toluol, Chlorbenzol oder Anisol.

Als Base können anorganische oder organische Basen verwendet werden. Beispielhaft seien hier genannt: Natriumhydroxid, Kaliumhydroxid, Natriumcarbonat, Natriumhydrogencarbonat, Kaliumcarbonat, Kaliumhydrogencarbonat, Triethylamin, Tributylamin, Morpholin, Piperidin, Pyridin, 2-Methyl-5-ethyl-pyridin oder Gemische dieser Basen. Bevorzugt verwendet man Natrium- oder Kaliumcarbonat. Besonders bevorzugt verwendet man Natriumcarbonat. Ebenfalls besonders bevorzugt verwendent man das Gemisch von Natriumcarbonat mit Natriumhydroxid.

Die Menge an Base richtet sich danach, ob man die Hydrochloride der Verbindungen der allgemeinen Formeln (IV'), (V') und (VI') oder die Verbindungen der allgemeinen Formeln (IV'), (V') und (VI') einsetzt. Setzt man die Hydrochloride der Verbindungen der allgemeinen Formeln (IV`), (V`) und (VI') ein, wird man mindestens zwei Moläquivalente Base verwenden, um die Hydrochloride *in situ* in die freien Verbindungen der allgemeinen Formeln (IV`), (V`) und (VI') zu überführen und anschließend die Acylierungsreaktion durchzuführen. Setzt man hingegen direkt die freien Verbindungen der allgemeinen Formeln (IV'), (V`) und (VI') ein, verwendet man mindestens ein Moläquivalente Base.

Das Säurechlorid der allgemeinen Formel (VII) kann in beliebigen Molverhältnissen, bezogen auf das Gemisch der Verbindungen (IV'), (V') und (VI'), eingesetzt werden. Üblicherweise verwendet man zwischen 0,9 und 2 Moläquivalente Säurechlorid, bevorzugt zwischen 0,95 und 1,3 Moläquivalente.

Die Reaktionstemperatur liegt zwischen -10 und 120°C, bevorzugt zwischen 0 und 100°C.

Die Reaktion kann grundsätzlich auch bei vermindertem oder erhöhtem Druck durchgeführt werden.

Die Aufarbeitung erfolgt nach bekannten Methoden der organischen Chemie, beispielsweise durch Filtration oder Extraktion.

Dritter Schritt (3) des erfindungsgemäßen Verfahrens (B): Die Verbindungen der allgemeinen Formeln (VIII'), (IX') und (X') werden in Gegenwart eines inerten Verdünnungsmittels und einer starken Base zu den Verbindungen der allgemeinen Formeln (XI), (XII') und (XIII) umgesetzt.

Als Verdünnungsmittel kommen beispielsweise in Frage: Toluol, ortho-, meta oder para-Xylol, Mesitylen, Chlorbenzol, ortho-Dichlorbenzol, Anisol, Acetonitril, Butyronitril, Tetrahydrofuran, 2-Methyl-tetrahydrofuran, Cyclopentyl-methyl-ether, Methyl-tertiärbutyl-ether, Tertiäramyl-methyl-ether, 1,4-Dioxan, N,N-Dimethylformamid, N,N-Dimethylacetamid, N-Methyl-pyrrolidon, Methanol, Ethanol, 1-Butanol, Tertiärbutanol oder Gemische dieser Lösungsmittel. Bevorzugt sind N,N-Dimethylformamid, N,N-Dimethylacetamid (DMAc), N-Methyl-pyrrolidon (NMP), Methanol, Tertiärbutanol, Chlorbenzol, ortho-Dichlorbenzol, Anisol oder Gemische dieser Lösungsmittel. Besonders bevorzugt sind DMAc, NMP, Toluol, Chlorbenzol und Anisol.

Als Basen können beispielsweise Natriumhydroxid, Kaliumhydroxid, Natriummethylat, Kaliummethylat, Natriumethylat, Kaliumethylat, Natrium-tertiärbutylat oder Kalium-tertiärbutylat verwendet werden. Bevorzugt sind Natriummethylat und Kalium-tertiärbutylat. Besonders bevorzugt verwendet wird Natriummethylat.

Die Basen werden in einer Menge von 0,9 bis 4 Moläquivalenten, bezogen auf die Verbindungen der allgemeinen Formeln (VIII`), (IX') und (X') eingesetzt. Bevorzugt verwendet man 1 bis 3,5 Moläquivalente.

Die Temperatur beträgt zwischen 20 und 170°C. Bevorzugt arbeitet man zwischen 40 und 150°C.

Die Isolierung der Verbindungen der allgemeinen Formeln (XI), (XII') und (XIII) nach Einstellen des pH-Wertes des Reaktionsgemisches auf einen Wert zwischen 0 und 8 erfolgt nach bekannten üblichen Methoden der organischen Chemie wie Filtration, Phasentrennung oder Extraktion.

Vierter Schritt (4) des erfindungsgemäßen Verfahrens (B): Das Gemisch der Verbindungen der allgemeinen Formeln (XI), (XII`) und (XIII) wird in Gegenwart eines inerten Verdünnungsmittels und einer Säure mit einem α,ω̅-Diol der allgemeinen Formel (XIV) zur Verbindung der allgemeinen Formel (XI) umgesetzt.

Als Verdünnungsmittel kommen beispielsweise in Frage: Dichlormethan, Toluol, ortho-, meta oder para-Xylol, Mesitylen, Chlorbenzol, ortho-Dichlorbenzol, Acetonitril, Butyronitril, Tetrahydrofuran, 2-Methyl-tetrahydrofuran, Cyclopentyl-methyl-ether, Methyl-tertiärbutyl-ether, Tertiäramyl-methyl-ether, 1,4-Dioxan, Anisol oder Gemische dieser Lösungsmittel. Bevorzugt sind Toluol, ortho-, meta oder para-Xylol, Chlorbenzol, Acetonitril, Butyronitril, 2-Methyl-tetrahydrofuran, Cyclopentyl-methyl-ether, Methyl-tertiärbutyl-ether, Tertiäramyl-methyl-ether oder Gemische dieser Lösungsmittel.

Das α,ω̅-Diol der allgemeinen Formel (XIV) wird in einer Menge von mindestens 1 Mol, bezogen auf 1 Mol der Verbindungen der allgemeinen Formeln (XII') und (XIII) eingesetzt. Es ist auch möglich, in einem beliebigen Überschuß an (α,ω̅-Diol der allgemeinen Formel (XIV) zu arbeiten und dieses somit gleichzeitig als Lösungsmittel zu verwenden.

Die vierte Stufe des erfindungsgemäßen Verfahrens wird in Gegenwart einer katalytischen Menge einer Säure durchgeführt. Als Säure kommen beispielsweise in Frage: Chlorwasserstoff, Schwefelsäure, Methansulfonsäure, Trifluormethansulfonsäure, para-Toluolsulfonsäure oder saure Ionenaustauscherharze wie beispielsweise Amberlite. Bevorzugt verwendet man Schwefelsäure oder para-Toluolsulfonsäure. Besonders bevorzugt verwendet man Schwefelsäure.

Die Säure wird in Mengen von 0,01 bis 20 Gewichtsprozent, bezogen auf die Verbindungen der allgemeinen Formeln (XII') und (XIII), eingesetzt. Bevorzugt sind 0,05 bis 10 Gewichtsprozent.

Die vierte Stufe des erfindungsgemäßen Verfahrens wird bei Temperaturen zwischen 20 und 150°C durchgeführt; bevorzugt zwischen 50 und 120°C.

Zur Erreichung eines möglichst weitgehenden Umsatzes kann es vorteilhaft sein, das entstehende Reaktionswasser zu entfernen, beispielsweise durch Destillation.

Die Isolierung der Verbindungen der allgemeine Formel (XI) erfolgt nach bekannten üblichen Methoden der organischen Chemie wie Filtration, Phasentrennung oder Extraktion.

Im Folgenden wird das erfindungsgemäße Verfahren (C) näher erläutert.

Erster Schritt (1) des erfindungsgemäßen Verfahrens (C): Die Reaktion von Verbindungen der allgemeinen Formel (III) mit einem Alkohol der allgemeinen Formel (XV) und Thionylchlorid zu den Hydrochloriden der Verbindungen der allgemeinen Formeln (IV`), (V`) und (VI') kann ohne Verdünnungsmittel oder gegebenenfalls in Gegenwart eines inerten Verdünnungsmittel wie beispielsweise Toluol, Chlorbenzol, 1,2-Dichlorbenzol, Heptan, Isooctan, Methylcyclohexan, Anisol oder Acetonitril durchgeführt werden. Bevorzugt arbeitet man ohne Verdünnungsmittel, d.h. der zur Veresterung verwendete Alkohol wird im Überschuß als Verdünnungsmittel eingesetzt.

Die Menge an Thionylchlorid kann in weiten Grenzen variiert werden. Üblicherweise arbeitet man mit 0,5 bis 5 Moläquivalenten Thionylchlorid, bezogen auf Verbindung der allgemeinen Formel (III). Bevorzugt verwendet man 0,9 bis 3 Moläquivalente Thionylchlorid. Besonders bevorzugt verwendet man 1,2 bis 3 Moläquivalente Thionylchlorid.

Die Reaktionstemperatur liegt zwischen -10 und 150°C, bevorzugt zwischen 0 und 120°C.

Die Reaktion kann grundsätzlich auch bei vermindertem oder erhöhtem Druck durchgeführt werden.

Die Aufarbeitung kann beispielsweise durch Abdestillieren des Alkohols und überschüssigen Thionylchlorids erfolgen. Auf diese Weise erhält man die Hydrochloride der Verbindungen der allgemeinen Formeln (IV`), (V`) und (VI`), die als solche in den nächsten Schritt des erfindungsgemäßen Verfahrens (C) eingesetzt werden können.

Es ist jedoch auch möglich, die Hydrochloride der Verbindungen der allgemeinen Formeln (IV'), (V') und (VI') durch Zusatz einer Base in die freien Aminosäureverbindungen der allgemeinen Formeln (IV`), (V`) und (VI') zu überführen und diese durch übliche Aufarbeitungsmethoden wie Filtration oder Extraktion zu isolieren.

Als Base können inerte anorganische und organische Basen verwendet werden wie beispielsweise Natriumcarbonat, Natriumhydrogencarbonat, Kaliumcarbonat, Kaliumhydrogencarbonat, Lithiumhydroxid, Natriumhydroxid, Kaliumhydroxid, Ammoniak, Triethylamin, Tributylamin, Pyridin oder 2-Methyl-5-ethyl-pyridin oder Gemische dieser Basen.. Bevorzugt wird Natriumcarbonat verwendet. Ebenfalls bevorzugt verwendet man das Gemisch von Natriumcarbonat mit Natriumhydroxid.

Die Menge an Base wird so gewählt, dass der Anteil an Hydrochlorid (HCl) im Gemisch der Hydrochloride der Verbindungen der allgemeinen Formeln (IV`), (V`) und (VI') neutralisiert wird.

Zweiter Schritt (2) des erfindungsgemäßen Verfahrens (C): Die Hydrochloride der Verbindungen der allgemeinen Formeln (IV'), (V') und (VI') oder die Verbindungen der allgemeinen Formeln (IV'), (V') und (VI') werden in Gegenwart eines inerten Verdünnungsmittels und einer Base mit einem Säurechlorid der allgemeinen Formel (VII) zu Verbindungen der allgemeinen Formeln (VIII`), (IX') und (X') umgesetzt.

Als Verdünnungsmittel können beispielsweise Dichlormethan, Toluol, Xylol, Chlorbenzol, 1,2-Dichlorbenzol, Heptan, Isooctan, Methylcyclohexan, Essigester, Acetonitril, Anisol, Tetrahydrofuran oder Butyronitril verwendet werden. Bevorzugt verwendet man Tetrahydrofuran, Anisol, Toluol, Xylol, Chlorbenzol oder Acetonitril. Besonders bevorzugt verwendet man Toluol, Chlorbenzol oder Anisol.

Als Base können anorganische oder organische Basen verwendet werden. Beispielhaft seien hier genannt: Natriumhydroxid, Kaliumhydroxid, Natriumcarbonat, Natriumhydrogencarbonat, Kaliumcarbonat, Kaliumhydrogencarbonat, Triethylamin, Tributylamin, Morpholin, Piperidin, Pyridin, 2-Methyl-5-ethyl-pyridin. Bevorzugt verwendet man Natrium- oder Kaliumcarbonat. Besonders bevorzugt verwendet man Natriumcarbonat. Ebenfalls besonders bevorzugt verwendet man das Gemisch von Natriumcarbonat mit Natriumhydroxid.

Die Menge an Base richtet sich danach, ob man die Hydrochloride der Verbindungen der allgemeinen Formeln (IV'), (V') und (VI') oder die Verbindungen der allgemeinen Formeln (IV'), (V') und (VI') einsetzt. Setzt man die Hydrochloride der Verbindungen der allgemeinen Formeln (IV`), (V`) und (VI') ein, wird man mindestens zwei Moläquivalente Base verwenden, um die Hydrochloride *in situ* in die freien Verbindungen der allgemeinen Formeln (IV`), (V`) und (VI') zu überführen und anschließend die Acylierungsreaktion durchzuführen. Setzt man hingegen direkt die freien Verbindungen der allgemeinen Formeln (IV'), (V`) und (VI') ein, verwendet man mindestens ein Moläquivalente Base.

Das Säurechlorid der allgemeinen Formel (VII) kann in beliebigen Molverhältnissen, bezogen auf das Gemisch der Verbindungen (IV'), (V') und (VI'), eingesetzt werden. Üblicherweise verwendet man zwischen 0,9 und 2 Moläquivalente Säurechlorid, bevorzugt zwischen 0,95 und 1,3 Moläquivalente.

Die Reaktionstemperatur liegt zwischen -10 und 120°C, bevorzugt zwischen 0 und 100°C.

Die Reaktion kann grundsätzlich auch bei vermindertem oder erhöhtem Druck durchgeführt werden.

Die Aufarbeitung erfolgt nach bekannten Methoden der organischen Chemie, beispielsweise durch Filtration oder Extraktion.

Dritter Schritt (3) des erfindungsgemäßen Verfahrens (C): Das Gemisch der Verbindungen der allgemeinen Formeln (VIII'), (IX') und (X') wird in Gegenwart eines inerten Verdünnungsmittels und einer Säure mit einem α,ω̅-Diol der allgemeinen Formel (XIV) zu Verbindung der allgemeinen Formel (XVI) umgesetzt.

Als Verdünnungsmittel kommen beispielsweise in Frage: Dichlormethan, Toluol, ortho-, meta oder para-Xylol, Mesitylen, Chlorbenzol, ortho-Dichlorbenzol, Acetonitril, Butyronitril, Tetrahydrofuran, 2-Methyl-tetrahydrofuran, Cyclopentyl-methyl-ether, Methyl-tertiärbutyl-ether, Tertiäramyl-methyl-ether, 1,4-Dioxan, Anisol oder Gemische dieser Lösungsmittel. Bevorzugt sind Anisol, Toluol, ortho-, meta oder para-Xylol, Chlorbenzol, Acetonitril, Butyronitril, 2-Methyl-tetrahydrofuran, Cyclopentyl-methyl-ether, Methyl-tertiärbutyl-ether, Tertiäramyl-methyl-ether oder Gemische dieser Lösungsmittel. Besonders bevorzugt sind Anisol, Toluol, Chlorbenzol.

Das α,ω̅-Diol der allgemeinen Formel (XIV) wird in einer Menge von mindestens 0.5 Mol, bezogen auf 1 Mol der Verbindungen der allgemeinen Formeln (VIII'), (IX') und (X') eingesetzt. Es ist auch möglich, in einem beliebigen Überschuß an (α,ω̅-Diol der allgemeinen Formel (XIV) zu arbeiten und dieses somit gleichzeitig als Lösungsmittel zu verwenden.

Die dritte Stufe des erfindungsgemäßen Verfahrens (C) wird in Gegenwart einer katalytischen Menge einer Säure durchgeführt. Als Säure kommen beispielsweise in Frage: Chlorwasserstoff, Schwefelsäure, Methansulfonsäure, Trifluormethansulfonsäure, para-Toluolsulfonsäure oder saure Ionenaustauscherharze wie beispielsweise Amberlite. Bevorzugt verwendet man Schwefelsäure, Salzsäure oder para-Toluolsulfonsäure. Besonders bevorzugt verwendet man Salzsäure oder para-Toluolsulfonsäure.

Die Säure wird in Mengen von 0,01 bis 20 Gewichtsprozent, bezogen auf die Verbindungen der allgemeinen Formeln (VIII'), (IX`) und (X`) eingesetzt. Bevorzugt sind 0,05 bis 10 Gewichtsprozent.

Die dritte Stufe des erfindungsgemäßen Verfahrens (C) wird bei Temperaturen zwischen 20 und 150°C durchgeführt; bevorzugt zwischen 50 und 140°C.

Zur Erreichung eines möglichst weitgehenden Umsatzes kann es vorteilhaft sein, das entstehende Reaktionswasser zu entfernen, beispielsweise durch Destillation.

Die Isolierung der Verbindung der allgemeinen Formel (XVI) erfolgt nach bekannten üblichen Methoden der organischen Chemie wie Filtration, Phasentrennung oder Extraktion.

Vierter Schritt (4) des erfindungsgemäßen Verfahrens (C): Die Verbindung der allgemeinen Formel (XVI) wird in Gegenwart eines inerten Verdünnungsmittels und einer starken Base zu der Verbindung der allgemeinen Formel (XI) umgesetzt.

Als Verdünnungsmittel kommen beispielsweise in Frage: Toluol, ortho-, meta oder para-Xylol, Mesitylen, Chlorbenzol, ortho-Dichlorbenzol, Acetonitril, Butyronitril, Tetrahydrofuran, 2-Methyl-tetrahydrofuran, Cyclopentyl-methyl-ether, Methyl-tertiärbutyl-ether, Tertiäramyl-methyl-ether, 1,4-Dioxan, N,N-Dimethylformamid, N,N-Dimethylacetamid, N-Methyl-pyrrolidon, Methanol, Ethanol, 1-Butanol, Tertiärbutanol, Anisol oder Gemische dieser Lösungsmittel. Bevorzugt sind N,N-Dimethylformamid, N,N-Dimethylacetamid, N-Methyl-pyrrolidon, Methanol, Tertiärbutanol, Anisol, Chlorbenzol, ortho-Dichlorbenzol oder Gemische dieser Lösungsmittel. Besonders bevorzugt sind DMAc, NMP, Xylol, Toluol, Chlorbenzol, Anisol. Ganz besonders bevorzugt sind DMAc, Toluol, Chlorbenzol, Anisol.

Als Basen können beispielsweise Natriummethylat, Kaliummethylat, Natriumethylat, Kaliumethylat, Natriumpropanolat, Kaliumpropanolat, Natrium-tertiärbutylat oder Kalium-tertiärbutylat verwendet werden. Bevorzugt sind Natriumhydroxid, Natriummethylat und Kalium-tertiärbutylat. Besonders bevorzugt verwendet wird Natriummethylat.

Die Basen werden in einer Menge von 0,9 bis 4 Moläquivalenten, bezogen auf die Verbindungen der allgemeinen Formel (XVI) eingesetzt. Bevorzugt verwendet man 1 bis 3,5 Moläquivalente.

Die Temperatur beträgt zwischen 20 und 170°C. Bevorzugt arbeitet man zwischen 40 und 150°C.

Die Isolierung der Verbindungen der allgemeinen Formel (XI) nach Einstellen des pH-Wertes des Reaktionsgemisches auf einen Wert zwischen 0 und 8 erfolgt nach bekannten üblichen Methoden der organischen Chemie wie Filtration, Phasentrennung oder Extraktion.

In einer bevorzugten Ausführungsform der Erfindung verwendet man im Verfahren C im zweiten, dritten und vierten Schritt Toluol als Lösungsmittel.

In einer bevorzugten Ausführungsform der Erfindung verwendet man im Verfahren C im zweiten und im dritten Schritt Toluol als Lösungsmittel und im vierten Schritt DMAc als Lösungsmittel.

In einer bevorzugten Ausführungsform der Erfindung verwendet man im Verfahren C im zweiten, dritten und vierten Schritt Chlorbenzol als Lösungsmittel.

In einer bevorzugten Ausführungsform der Erfindung verwendet man im Verfahren C im zweiten und im dritten Schritt Chlorbenzol als Lösungsmittel und im vierten Schritt DMAc als Lösungsmittel.

In einer bevorzugten Ausführungsform der Erfindung verwendet man im Verfahren C im zweiten, dritten und vierten Schritt Anisol als Lösungsmittel.

In einer bevorzugten Ausführungsform der Erfindung verwendet man im Verfahren C im ersten, zweiten, dritten und vierten Schritt Chlorbenzol als Lösungsmittel.

Ebenfalls Gegenstand der vorliegenden Erfindung sind neue Verbindungen der allgemeinen Formel (V`) 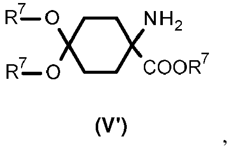 dadurch gekennzeichnet, dass der Rest
- R⁷: für n-Propyl steht.

Ebenfalls Gegenstand der vorliegenden Erfindung sind neue Verbindungen der allgemeinen Formel (VI') 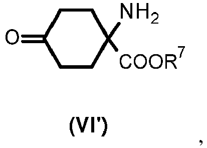 in welcher
- R⁷: für n-Propyl steht.

Ebenfalls Gegenstand der vorliegenden Erfindung sind neue Verbindungen der allgemeinen Formel (IX') 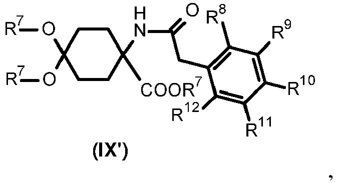 in welcher
- R⁷: für n-Propyl,
- R⁸: für Methyl,
- R⁹: für Wasserstoff,
- R¹⁰: für Chlor,
- R¹¹: für Wasserstoff
und
- R¹²: für Methyl steht.

Ebenfalls Gegenstand der vorliegenden Erfindung sind neue Verbindungen der allgemeinen Formel (X') 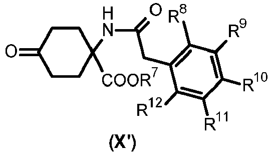 in welcher
- R⁷: für n-Propyl,
- R⁸: für Methyl,
- R⁹: für Wasserstoff,
- R¹⁰: für Chlor,
- R¹¹: für Wasserstoff
und
- R¹²: für Methyl steht.

Ebenfalls Gegenstand der vorliegenden Erfindung sind neue Verbindungen der allgemeinen Formel (XII') 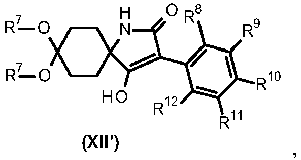 in welcher
- R⁷: für n-Propyl,
- R⁸: für Methyl,
- R⁹: für Wasserstoff,
- R¹⁰: für Chlor,
- R¹¹: für Wasserstoff und
- R¹²: für Methyl steht.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Die vorliegende Erfindung wird durch die folgenden Beispiele näher veranschaulicht ohne durch sie eingeschränkt zu sein.

### Beispiele

### Beispiel 1 (nicht erfindungsgemäß)

### Methyl-8-amino-1,4-dioxaspiro[4.5]decan-8-carboxylat, Methyl-1-amino-4,4-dimethoxycyclohexan-carboxylat und Methyl-1-amino-4-oxocyclohexancarboxylat

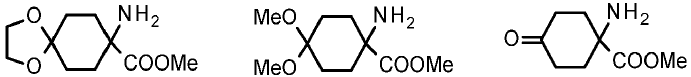

Man legt 29,1 g [0,145 Mol] 8-Amino-1,4-dioxaspiro[4.5]decan-8-carbonsäure in 160 g Methanol vor. Bei 0 - 5°C werden unter Kühlung 34,4 g [0,289 Mol] Thionylchlorid zudosiert. Man läßt auf 20°C erwärmen, rührt für 16 Stunden bei 20°C und dann noch für 4 Stunden unter Rückfluß (64°C). Methanol und Thionylchlorid werden unter vermindertem Druck abdestilliert. Der Rückstand wird mit Natronlauge (32%) auf pH 8 gestellt und zweimal mit je 100 mL Methyl-tertiärbutyl-ether (MTBE) extrahiert. Die vereinigten organischen Phasen werden über Natriumsulfat getrocknet und unter vermindertem Druck eingeengt. Man erhält 19,5 g Rückstand, der nach ¹H- und ¹³C-NMR-Analysen aus den drei Titelverbindungen besteht.

Zusammensetzung nach quantitativem ¹H-NMR: 61% Methyl-8-amino-1,4-dioxaspiro[4.5]decan-8-carboxylat; 9% Methyl-1-amino-4,4-dimethoxycyclohexancarboxylat; 21% Methyl-1-amino-4-oxocyclohexancarboxylat.

Methyl-8-amino-1,4-dioxaspiro[4.5]decan-8-carboxylat: ¹³C-NMR (150 MHz, d-DMSO): δ = 30,2 (CH₂), 32,5 (CH₂), 51,8 (***Me***-OCO), 55,9 (***C***(NH₂)COOMe), 63,7 (O-***C***H₂-C), 63,8 (O-***C***H₂-C), 107,8 (***C***-OCCO-), 177,6 (***C***(=O)OMe) ppm.

Methyl-1-amino-4,4-dimethoxycyclohexancarboxylat: ¹³C-NMR (150 MHz, d-DMSO): δ = 27,7 (CH₂), 31,5 (CH₂), 47,0 (***Me***O-C(OMe)), 47,1 (***Me***O-C(OMe)), 51,8 (***M*e**-OCO), 56,2 (***C***(NH₂)COOMe), 99,2 (***C***(OMe)₂), 177,6 (***C***(=O)OMe) ppm.

Methyl-1-amino-4-oxocyclohexancarboxylat: ¹³C-NMR (150 MHz, d-DMSO): δ = 34,1 (CH₂), 36,6 (CH₂), 52,0 (***Me***-OCO), 55,7 (***C***(NH₂)COOMe), 177,1 (***C***(=O)OMe), 210,1 (C-***C***(=O)-C) ppm.

### Beispiel 2 (nicht erfindungsgemäß)

### Ethyl-8-amino-1,4-dioxaspiro[4.5]decan-8-carboxylat und Ethyl-1-amino-4-oxocyclohexancarboxylat

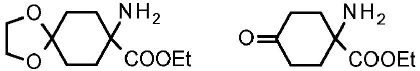

Man verfährt wie in Beispiel 1 beschrieben mit der Änderung, dass Ethanol anstatt Methanol verwendet wird. Man erhält 29 g Gemisch, das nach ¹H- und ¹³C-NMR-Analysen aus den beiden Titelverbindungen besteht.

Zusammensetzung nach quantitativem ¹H-NMR: 83,6% Ethyl-8-amino-1,4-dioxaspiro[4.5]decan-8-carboxylat; 16,7% Ethyl-1-amino-4-oxocyclohexancarboxylat.

Ethyl-8-amino-1,4-dioxaspiro[4.5]decan-8-carboxylat: ¹³C-NMR (150 MHz, d-DMSO): δ = 14,2 (***C***H₃-CH₂O(CO)), 30,3 (CH₂), 32,5 (CH₂), 55,9 (***C***(NH₂)COOMe), 60,3 (CH₃-***C***H₂O(CO)), 63,8 (O-***C***H₂-C), 107,8 (***C***-OCCO-), 177,1 (***C***(=O)OMe) ppm.

Ethyl-1-amino-4-oxocyclohexancarboxylat: ¹³C-NMR (150 MHz, d-DMSO): δ = 14,2 (***C***H₃-CH₂O(CO)), 34,2 (CH₂), 36,6 (CH₂), 55,6 (***C***(NH₂)COOMe), 60,5 (CH₃-***C***H₂O(CO)), 176,6 (***C***(=O)OMe), 210,2 ((C-***C***(=O)-C) ppm.

### Beispiel 3

### n-Propyl-8-amino-1,4-dioxaspiro[4.5]decan-8-carboxylat

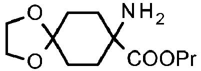

Man verfährt wie in Beispiel 1 beschrieben mit der Änderung, dass 1-Propanol anstatt Methanol verwendet wird. Man erhält 29,5 g der Titelverbindung.

Reinheit nach quantitativem ¹H-NMR: 90%.

n-Propyl-8-amino-1,4-dioxaspiro[4.5]decan-8-carboxylat: ¹³C-NMR (150 MHz, d-DMSO): δ = 10,4 (***C***H₃-CH₂CH₂O(CO)), 21,7 (CH₃-***C***H₂CH₂O(CO)), 30,3 (CH₂), 32,5 (CH₂), 56,0 (***C***(NH₂)COOMe), 63,8 (O-***C***H₂-C), 65,7 (CH₃CH₂-***C***H₂O(CO)), 107,8 (***C***-OCCO-), 177,1 (***C***(=O)OMe) ppm.

### Beispiel 4

### n-Propyl-8-amino-1,4-dioxaspiro[4.5]decan-8-carboxylat, n-Propyl-1-amino-4,4-dipropoxycyclohexan-carboxylat und n-Propyl-1-amino-4-oxocyclohexancarboxylat

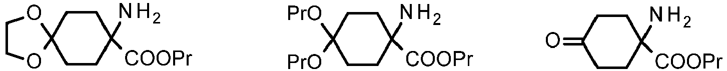

Man legt 99 g [0,492 Mol] 8-Amino-1,4-dioxaspiro[4.5]decan-8-carbonsäure in 200 g 1-Propanol vor. Bei 0 - 5°C werden unter Kühlung innerhalb einer Stunde 76,1 g [0,64 Mol] Thionylchlorid zudosiert. Man läßt auf 20°C erwärmen, rührt für 16 Stunden bei 20°C und dann noch für 6 Stunden unter Rückfluß. Propanol und Thionylchlorid werden unter vermindertem Druck abdestilliert. Der Rückstand wird mit wässriger Sodalösung auf pH 8 gestellt und zweimal mit je 150 mL Methyl-tertiärbutyl-ether (MTBE) extrahiert. Die vereinigten organischen Phasen werden mit 50 mL Wasser gewaschen, über Natriumsulfat getrocknet und unter vermindertem Druck eingeengt. Man erhält 100 g Rückstand, der nach ¹H- und ¹³C-NMR-Analysen aus den drei Titelverbindungen besteht.

Zusammensetzung nach quantitativem ¹H-NMR: 69% n-Propyl-8-amino-1,4-dioxaspiro[4.5]decan-8-carboxylat, 16% n-Propyl-1-amino-4,4-dipropoxycyclohexancarboxylat und 10% n-Propyl-1-amino-4-oxocyclohexancarboxylat.

n-Propyl-8-amino-1,4-dioxaspiro[4.5]decan-8-carboxylat: ¹³C-NMR (150 MHz, d6-DMSO): δ = 10,4 (***C***H₃-CH₂CH₂O(CO)), 21,7 (CH₃-***C***H₂CH₂O(CO)), 30,3 (CH₂), 32,5 (CH₂), 56,0 (***C***(NH₂)COOMe), 63,8 (O-***C***H₂-C), 65,7 (CH₃CH₂-***C***H₂O(CO)), 107,8 (***C***-OCCO-), 177,1 (***C***(=O)OMe) ppm.

n-Propyl 1-amino-4,4-dipropoxy-cyclohexanecarboxylat: ¹³C-NMR (150 MHz, d6-DMSO): δ = 10,5 (***C***H₃-CH₂CH₂O(CO)), 11,0/11,1 (C(OCH₂CH₂***C***H₃)₂), 21,7 (CH₃-***C***H₂CH₂O(CO)), 23.0 (2xCH₂), 30,3 (2xCH₂), 32,6 (2xCH₂), 56,0 (***C***(NH₂)COOPr), 60.7/60.8 (C(O_{C}H₂CH₂CH₃)₂), 65,7 (CH₃CH₂-*C*H₂O(CO)), 98.9 (***C***(OCH₂CH₂CH₃)₂), 177,2 (C-***C***O-OPr) ppm.

n-Propyl 1-amino-4-oxo-cyclohexanecarboxylat: ¹³C-NMR (150 MHz, d6-DMSO): δ = 10,4 (***C***H₃-CH₂CH₂O(CO)), 21,5 (CH₃-***C***H₂CH₂O(CO)), 29.6 (2xCH₂), 35,8 (2xCH₂), 57,3 (***C***(NH₂)COOPr), 67,9 (CH₃CH₂-***C***H₂O(CO)), 170,9 (C-***C***O-OPr), 207.8 (C-(**C**=O)-C) ppm.

### Beispiel 5 (nicht erfindungsgemäß)

### n-Butyl-8-amino-1,4-dioxaspiro[4.5]decan-8-carboxylat und n-Butyl-1-amino-4,4-dibutoxycyclohexan-carboxylat

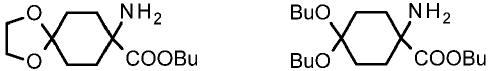

Man verfährt wie in Beispiel 1 beschrieben mit der Änderung, dass 1-Butanol anstatt Methanol verwendet wird. Man erhält 39 g Gemisch, das nach ¹H- und ¹³C-NMR-Analysen aus den beiden Titelverbindungen besteht.

Zusammensetzung nach quantitativem ¹H-NMR: 69% n-Butyl-8-amino-1,4-dioxaspiro[4.5]decan-8-carboxylat und 23% n-Butyl-1-amino-4,4-dibutoxycyclohexancarboxylat.

n-Butyl-8-amino-1,4-dioxaspiro[4.5]decan-8-carboxylat: ¹³C-NMR (150 MHz, d6-DMSO): δ = 13,7 (***C***H₃-CH₂CH₂CH₂O(CO)), 18,8 (CH₃-***C***H₂CH₂CH₂O(CO)), 30,4 (CH₃-CH₂***C***H₂CH₂O(CO)), 30,4 (***C***H₂), 32,6 (CH₂), 56,0 (***C***(NH₂)COOMe), 63,7 (O-***C***H₂-C), 63,9 (CH₃CH₂CH₂-***C***H₂O(CO)), 107,8 (***C***-OCCO-), 177,1 (***C***(=O)OMe) ppm.

n-Butyl-1-amino-4,4-dibutoxycyclohexancarboxylat: ¹³C-NMR (150 MHz, d6-DMSO): δ = 13,7 (***C***H₃-CH₂CH₂CH₂O(CO)), 14,0 (***C***H₃CH₂CH₂CH₂O-C(OBu), 18,8 (CH₃-***C***H₂CH₂CH₂O(CO)), 19,3 (CH₃***C***H₂CH₂CH₂O-C(OBu), 28,7 (***C***H₂), 30,4 (CH₃-CH₂***C***H₂CH₂O(CO)), 31,6 (***C***H₂), 31,9 (CH₃CH₂***C***H₂CH₂O-C(OBu), 56,2 (***C***(NH₂)COOBu), 58,8 (CH₃CH₂CH₂***C***H₂O-C(OBu), 63,8 (CH₃-*C*H₂CH₂***C***H₂O(CO)), 98,9 (***C***(OBu)₂), 177,2 (***C***(=O)OBu) ppm.

### Beispiel 6

### n-Propyl-8-[2-(4-chlor-2,6-dimethylphenyl)acetamido]-1,4-dioxaspiro[4.5]decan-8-carboxylat

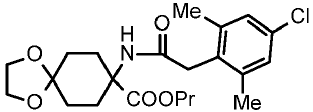

In 70 g 20%iger wässriger Natriumhydrogencarbonatlösung werden 27 g [0,111 Mol] n-Propyl-8-amino-1,4-dioxaspiro[4.5]decan-8-carboxylat (Verbindung aus Beispiel 3) vorgelegt. Bei ca. 5°C wird eine Lösung von 24,1 g [0,111 Mol] (4-Chlor-2,6-dimethylphenyl)acetylchlorid in 20,3 g Toluol innerhalb von etwa 2 Stunden zudosiert. Während dieser Dosierung gibt man noch 35 g Wasser und 50 g Toluol zum Reaktionsgemisch. Man rührt nach Beendigung der Dosierung noch 1 Stunde bei 20°C, saugt den Feststoff ab, wäscht ihn mit Wasser und trocknet ihn. Man erhält 29,5 g der Titelverbindung.

Reinheit nach quantitativem ¹H-NMR: 92%.

¹³C-NMR (150 MHz, d6-DMSO): δ = 10,4 (***C***H₃-CH₂CH₂O(CO)), 19,9 (***C***H₃-Ar), 21,7 (CH₃-***C***H₂CH₂O(CO)), 29,9 (CH₂), 30,2 (CH₂), 35,2 (N-CO-***C***H₂-Ar), 57,5 (***C***(NHCO)COOPr), 63,8 (O-***C***H₂***C***H₂O), 65,9 (CH₃CH₂-***C***H₂O(CO)), 107,3 (***C***(OCH₂CH₂O)), 127,1 (***C***_{Ar}-H), 130,4 (***C***_{Ar}-Cl), 133,0 (COCH₂-***C***_{Ar}), 139,6 (***C***_{Ar}-Me), 169,6 (N-***C***O-CH₂), 173,7 (C-***C***O-OPr) ppm.

### Nachfällung:

### n-Propyl-1-[2-(4-chlor-2,6-dimethylphenyl)acetamido]-4-oxocyclohexancarboxylat

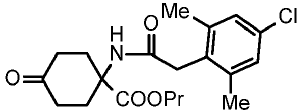

n-Propyl-1-[2-(4-chlor-2,6-dimethylphenyl)acetamido]-4-oxocyclohexancarboxylat: ¹³C-NMR (150 MHz, d6-DMSO): δ = 10,4 (***C***H₃-CH₂CH₂O(CO)), 19,9 (***C***H₃-Ar), 21,7 (CH₃-***C***H₂CH₂O(CO)), 31,5 (CH₂), 35,2 (N-CO-***C***H₂-Ar), 36,4 (CH₂), 57,2 (***C***(NHCO)COOPr), 66,2 (CH₃CH₂-***C***H₂O(CO)), 127,0 (***C***_{Ar}-H), 130,4 (***C***_{Ar}-Cl), 132,9 (COCH₂-***C***_{*A*r}), 139,7 (***C***_{Ar}-Me), 170,0 (N-***C***O-CH₂), 173,1 (C-***C***O-OPr), 209,1 (C-***C***O-C) ppm.

### n-Propyl 1-[[2-(4-chloro-2,6-dimethyl-phenyl)acetyl]amino]-4,4-dipropoxy-cyclohexanecarboxylat

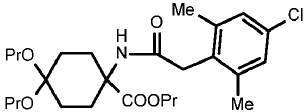

¹³C-NMR (150 MHz, d6-DMSO): δ = 10,4 (***C***H₃-CH₂CH₂O(CO)), 11,0 (2x C(OCH₂CH₂***C***H₃)₂), 20.1 (2x Aryl-**C**H₃), 21,6 (CH₃-***C***H₂CH₂O(CO)), 22,9/23.0 (2xCH₂), 28,6 (2xCH₂), 28,9 (2xCH₂), 35.2 (Aryl-**C**H₂-(CO)N), 57,8 (***C***(NH₂)COOPr), 60.8/60.9 (C(O***C***H₂CH₂CH₃)₂), 65,8 (CH₃CH₂-***C***H₂O(CO)), 98.5 (***C***(OCH₂CH₂CH₃)₂), 126,6 (2x ***C***_{Ar}-H), 128,8 (***C***_{Ar}-Cl), 136,3 (COCH₂-***C**_{Ar}*), 138,9 (2x ***C***_{Ar}-Me), 169,4 (N-***C***O-CH₂), 173,7 (C-***C***O-OPr) ppm.

### Beispiel 7 (nicht erfindungsgemäß)

### Methyl-8-amino-1,4-dioxaspiro[4.5]decan-8-carboxylat und Methyl-1-amino-4,4-dimethoxycyclohexan-carboxylat

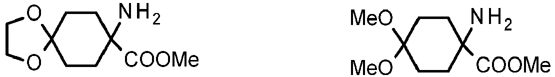

Man legt 2748 g [11,56 Mol] 8-Amino-1,4-dioxaspiro[4.5]decan-8-carbonsäure-Hydrochlorid in 50 L Methanol vor und dosiert dann innerhalb 1 Stunde bei 5 - 10°C 2050 g [17,23 Mol] Thionylchlorid zu. Man rührt 48 Stunden bei 40 - 45°C, kühlt danach auf 5°C ab, filtriert und wäscht den Feststoff mit 3 L Methanol nach. Das Filtrat wird unter vermindertem Druck eingeengt. Der erhaltenen Rückstand wird in einer Lösung von 1900 g Kaliumcarbonat in 8 L Wasser verrührt und fünfmal mit je 8 L Methylenchlorid extrahiert. Die vereinigten organischen Phasen werden über Natriumsulfat getrocknet und unter vermindertem Druck eingeengt. Man erhält 2240 g Öl, das nach GC/MS-Analyse 65,4% Methyl-8-amino-1,4-dioxaspiro[4.5]decan-8-carboxylat und 31,1% Methyl-1-amino-4,4-dimethoxycyclohexancarboxylat enthält.

### Beispiel 8 (nicht erfindungsgemäß)

Methyl-8-[2-(4-chlor-2,6-dimethylphenyl)acetamido]-1,4-dioxaspiro[4.5]decan-8-carboxylat, Methyl-1-[2-(4-chlor-2,6-dimethylphenyl)acetamido]-4,4-dimethoxycyclohexancarboxylat und Methyl-1-[2-(4-chlor-2,6-dimethylphenyl)acetamido]-4-oxocyclohexancarboxylat

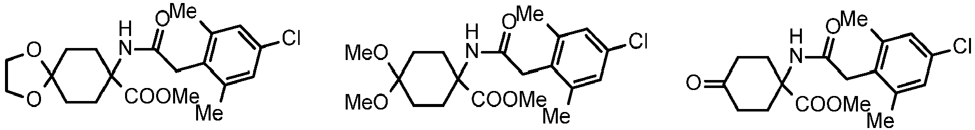

Man löst 100 g einer Mischung aus 67,5% Methyl-8-amino-1,4-dioxaspiro[4.5]decan-8-carboxylat [0,313 Mol], 30,1% Methyl-1-amino-4,4-dimethoxycyclohexancarboxylat [0,138 Mol] und 0,5% Methyl-1-amino-4-oxocyclohexancarboxylat [0,003 Mol] in 1,44 L Acetonitril. Unter Rühren gibt man 121 g Kaliumcarbonat und destilliert dann zur azeotropen Trocknung etwa 90 ml wasserfeuchtes Acetonitril ab. Danach wird auf 5°C gekühlt und innerhalb von 2 Stunden eine Lösung von 95,5 g [0,44 Mol] (4-Chlor-2,6-dimethylphenyl)acetylchlorid zugetropft. Man rührt dann noch für 2 Stunden bei 5°C und 16 Stunden bei 20°C, rührt das Reaktionsgemisch danach in 6,6 L Wasser ein, filtriert, wäscht den Feststoff mit 0,7 L Wasser, und trocknet ihn. Es resultieren 165 g weißer Feststoff, der nach GC/MS-Analyse zu 69,2% aus Methyl-8-[2-(4-chlor-2,6-dimethylphenyl)acetamido]-1,4-dioxaspiro[4.5]decan-8-carboxylat, 20,1% Methyl-1-[2-(4-chlor-2,6-dimethylphenyl)acetamido]-4,4-dimethoxycyclohexancarboxylat und 7,8% Methyl-1-[2-(4-chlor-2,6-dimethylphenyl)acetamido]-4-oxocyclohexancarboxylat besteht.

### Beispiel 9 (nicht erfindungsgemäß)

### Methyl-8-[2-(4-chlor-2,6-dimethylphenyl)acetamido]-1,4-dioxaspiro[4.5]decan-8-carboxylat, Methyl-1-[2-(4-chlor-2,6-dimethylphenyl)acetamido]-4,4-dimethoxycyclohexancarboxylat und Methyl-1-[2-(4-chlor-2,6-dimethylphenyl)acetamido]-4-oxocyclohexancarboxylat

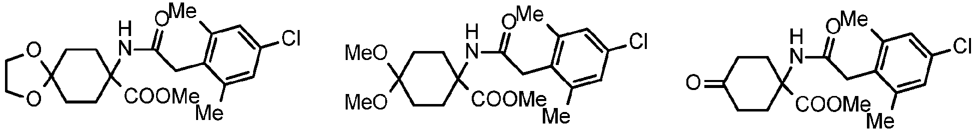

Man legt 5 g 8-[2-(4-Chlor-2,6-dimethylphenyl)acetamido]-1,4-dioxaspiro[4.5]decan-8-carbonsäure einer Reinheit von 84,6% (4,23 g, 11,1 mMol) in 50 mL Methanol vor, gibt 0,11 g konz. Schwefelsäure hinzu und erhitzt für 16 Stunden unter Rückfluß. Anschließend wird das Reaktionsgemisch im Vakuum eingeengt. Man erhält 5,5 g Rückstand, der lt. GC/MS-Analyse zu 57,6% aus Methyl-8-[2-(4-chlor-2,6-dimethylphenyl)acetamido]-1,4-dioxaspiro[4.5]decan-8-carboxylat und 35,2% Methyl-1-[2-(4-chlor-2,6-dimethylphenyl)acetamido]-4,4-dimethoxycyclohexancarboxylat besteht.

### Beispiel 10

### 11-(4-Chlor-2,6-dimethylphenyl)-12-hydroxy-1,4-dioxa-9-azadispiro[4.2.48.25]tetradec-11-en-10-on, 3-(4-Chlor-2,6-dimethylphenyl)-4-hydroxy-8,8-dimethoxy-1-azaspiro[4.5]dec-3-en-2-on und 3-(4-Chlor-2,6-dimethylphenyl)-4-hydroxy-1-azaspiro[4.5]dec-3-en-2,8-dion

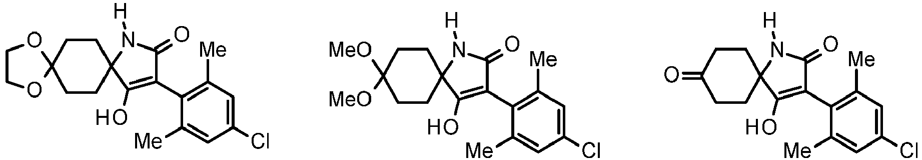

Man löst 5491 g [48,934 Mol] KOtBu in 31 L Dimethylformamid (DMF) und gibt dann innerhalb von 2 Stunden portionsweise 6550 g einer Mischung aus 68,3% Methyl-8-[2-(4-chlor-2,6-dimethylphenyl)-acetamido]-1,4-dioxaspiro[4.5]decan-8-carboxylat, 16,8% Methyl-1-[2-(4-chlor-2,6-dimethylphenyl)-acetamido]-4,4-dimethoxycyclohexancarboxylat und 12,1% Methyl-1-[2-(4-chlor-2,6-dimethylphenyl)-acetamido]-4-oxocyclohexancarboxylat hinzu, wobei sich das Reaktionsgemisch auf 40°C erwärmt. Man rührt noch 3 Stunden bei 40°C und über Nacht bei Raumtemperatur, destilliert 26 L DMF im Vakuum ab, versetzt den Rückstand mit 75 L Eiswasser, gibt 4 L Eisessig hinzu und rührt über Nacht bei 15°C. Der ausgefallene Feststoff wird abgesaugt, dreimal mit je 5 L Wasser gewaschen und getrocknet. Es resultieren 5720 g folgender Zusammensetzung lt. HPLC-Analyse:
71,8% 11-(4-Chlor-2,6-dimethylphenyl)-12-hydroxy-1,4-dioxa-9-azadispiro[4.2.48.25]tetradec-11-en-10-on; 13,4% 3-(4-Chlor-2,6-dimethylphenyl)-4-hydroxy-8,8-dimethoxy-1-azaspiro[4.5]dec-3-en-2-on und 8,3% 3-(4-Chlor-2,6-dimethylphenyl)-4-hydroxy-1-azaspiro[4.5]dec-3-en-2,8-dion.

### Beispiel 11

### 11-(4-Chlor-2,6-dimethylphenyl)-12-hydroxy-1,4-dioxa-9-azadispiro[4.2.48.25]tetradec-11-en-10-on

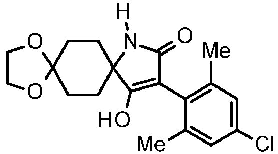

Die 5720 g Produktgemisch aus Beispiel 10 werden in 27 L Ethylenglykol suspendiert und mit 95 g para-Toluolsulfonsäure versetzt. Man erhitzt unter Rühren für zwei Stunden auf ca. 130°C (155°C Badtemperatur). Anschließend werden bei gleicher Badtemperatur 4 L Acetonitril zudosiert, wodurch die Innentemperatur auf ca. 111°C absinkt. Man rührt drei Stunden bei gleicher Badtemperatur, wobei das Acetonitril zunächst bei Normaldruck, im weiteren Verlauf bei einem bis etwa 200 mbar vermindertem Druck abdestilliert wird bis die Innentemperatur wieder ca. 130°C beträgt. Es wird über Nacht bei 130°C gerührt, dann auf Raumtemperatur abgekühlt, der Feststoff abgesaugt, für eine Stunde in 30 L Wasser verrührt, erneut abgesaugt, mit 10 L Wasser gewaschen und getrocknet. Man erhält 5080 g hellbeigen Feststoff, der lt. HPLC-Analyse zu 99,3% aus der Titelverbindung besteht.

### Beispiel 12

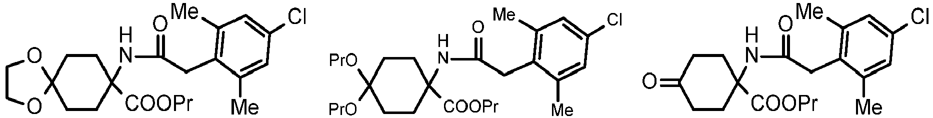

60g [0,296 Mol] 8-Amino-1,4-dioxaspiro[4.5]decan-8-carbonsäure (99%ig) werden in 154g 1-Propanol vorgelegt, die Mischung auf 80-85°C erwärmt und 24.8g Thionylchlorid mit 0.5ml/min zudosiert. Nach beendeter Dosierung wird 3h bei 90°C nachgerührt, dann abgekühlt auf 40-45°C. Bei etwa 30mbar werden 89g Destillat entfernt, anschließend wird das Vakuum gebrochen und auf 5-10°C abgekühlt. Es wird nun eine Mischung aus 9.3g NaOH und 61.9g Na₂CO₃ gelöst in 300g Wasser zudosiert, so dass die Innentemperatur stets unter 10°C bleibt. Zur Reaktionsmischung wird nun 128.6g 2-(4-Chlor-2,6-dimethyl-phenyl)acetylchlorid in THF (42.2%ige Lösung) mit 2.5ml/min dosiert. Es werden 200g THF nachgesetzt und mit verdünnter HCl auf pH 7-8 gestellt. Die organische Phase wird abgetrennt und am Rotationsverdampfer eingeengt. Man erhält 125g eines Gemischs aus n-Propyl 8-[[2-(4-chlor-2,6-dimethyl-phenyl)acetyl]amino]-1,4-dioxaspiro[4.5]decane-8-carboxylat (65%)
/ n-Propyl 1-[[2-(4-chlor-2,6-dimethyl-phenyl)acetyl]amino]-4,4-dipropoxy-cyclohexanecarboxylat (8%)
/ n-Propyl 1-[[2-(4-chlor-2,6-dimethyl-phenyl)acetyl]amino]-4-oxo-cyclohexanecarboxylat (13%). Ausbeute: 86%.

### Beispiel 13

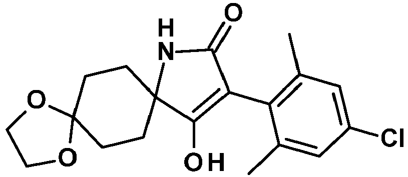

20g [0.041mol] des isolierten Rohproduktes aus Beispiel 12 werden in 60g Xylol, 3.9g Ethandiol und 0.3g para-Toluolsulfonsäure bei 130°C über 4h gerührt, weitere 0.9g der para-Toluolsulfonsäure sowie 3.9g Ethandiol werden nachgesetzt und für weitere 8 h refluxiert. Danach wird das Xylol am Rotationsverdampfer abdestilliert und der Rückstand in 80g DMAc aufgenommen. Das DMAc wird andestilliert und nach der Entfernung von 25g Destillat werden 8g Natriummethanolat (30%ige Lösung in Methanol) bei 110°C Innentemperatur addiert. Es wird erneut andestilliert bei 250mbar und etwa 100-110°C Innentemperatur, danach wird auf 80°C abgekühlt und 50g Wasser addiert, gefolgt von 10g Essigsäure. Nach Abkühlen auf Raumtemperatur wird die Suspension abgesaugt und mit 30g Wasser gewaschen. Man erhalt 9.5g 2-(4-Chlor-2,6-dimethyl-phenyl)-1-hydroxy-9,12-dioxa-4-azadispiro[4.2.48.25]tetradec-1-en-3-on in 64% Ausbeute und 97% Reinheit.

### Beispiel 14

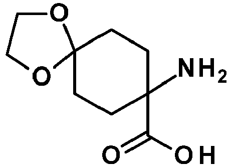

550g Natrium-8-amino-1,4-dioxaspiro[4.5]decan-8-carboxylat (~78% Reinheit) werden in 724g Wasser bei Raumtemperatur vorgelegt. Zu der Mischung wird 20%ige HCl langsam addiert bis der pH-Wert bei 5-6 liegt. Die Suspension wird abgesaugt, der Nutschkuchen mit 212,6g Wasser gewaschen und anschließend getrocknet.
Ausbeute: 356,7g (92%) 8-Amino-1,4-dioxaspiro[4.5]decan-8-carbonsäure, Reinheit 94% nach quant. NMR
¹H-NMR (600 MHz, d-D₂O + 1 drop NaOD): δ = 1.55-1.60 (m, 2H), 1.69-1.73 (m, 2H), 1.77-1.82 (m, 2H), 2.00-2.04 (m, 2H), 4,021 (s, 4H) ppm.

### Beispiel 15

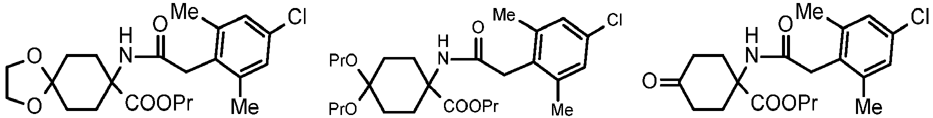

370,7g [0,296 Mol] 8-Amino-1,4-dioxaspiro[4.5]decan-8-carbonsäure (96.7%ig) werden in 1178g 1-Propanol vorgelegt, die Mischung auf 80-85°C erwärmt und 233g Thionylchlorid innerhalb von 2h zudosiert. Nach beendeter Dosierung wird 3h bei 90°C nachgerührt und die ~22-25%(w/w) Lösung für Folgereaktionen verwendet.

### Beispiel 16

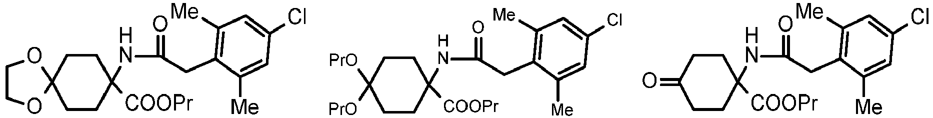

235.3g einer Lösung, die analog zu Beispiel 15 hergestellt wurde, wird bei 25-30°C Innentemperatur und 25mbar andestilliert. Nach Entfernung von 128.9g Destillat wird das Vakuum mit Stickstoff gebrochen und der Ansatz auf Normaldruck gebracht. Nach Abkühlen der Mischung auf 0-5°C werden 43.8g Na₂CO₃, gelöst in 400g Wasser, langsam zudosiert, so dass die Temperatur stets unter 5°C bleibt. Nach beendeter Dosierung werden 121.9g 2-(4-Chlor-2,6-dimethyl-phenyl)acetylchlorid (39.5%ig in Toluol, 0.85eq) in einer Stunde addiert, so dass die Innentemperatur 5°C nicht übersteigt. Der Ansatz wird 1h bei 0-5°C nachgerührt und anschließend auf 73°C erwärmt. Die organische Phase wird abgetrennt und am Rotationsverdampfer eingeengt. Man erhält 98.8g eines blassgelben Feststoffes aus 8-[[2-(4-Chlor-2,6-dimethyl-phenyl)acetyl]amino]-1,4-dioxaspiro[4.5]decan-8-carbonsäure-propylestere (84%) / 1-[[2-(4-Chloro-2,6-dimethyl-phenyl)acetyl]amino]-4,4-dipropoxy-cyclohexancarbonsäure-propylester (7%) / 1-[[2-(4-Chlor-2,6-dimethyl-phenyl)acetyl]amino]-4-oxo-cyclohexancarbonsäure-propylestere (7%). Die Ausbeute ist quantitativ.

### Beispiel 17

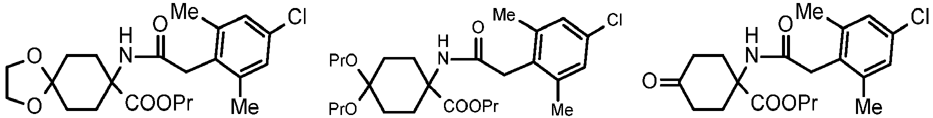

232.4g einer Lösung, die analog zu Beispiel 15 hergestellt wurde, wird bei 25-30°C Innentemperatur und 25mbar andestilliert. Nach Entfernung von 126.8g Destillat wird das Vakuum mit Stickstoff gebrochen und der Ansatz auf Normaldruck gebracht. Nach Abkühlen der Mischung auf 0-5°C werden 43.7g Na₂CO₃ gelöst in 400g Wasser langsam zudosiert, so dass die Temperatur stets unter 5°C bleibt. Nach beendeter Dosierung werden nun 133.6g 2-(4-Chlor-2,6-dimethyl-phenyl)acetylchlorid (39.5%ig in Toluol) in einer Stunde addiert, so dass die Innentemperatur 5°C nicht übersteigt. Der Ansatz wird 3h bei 0-5°C nachgerührt und anschließend auf 75°C erwärmt. Die organische Phase wird abgetrennt und am Rotationsverdampfer eingeengt. Man erhält 103,5g eines blassgelben Feststoffes aus 8-[[2-(4-Chlor-2,6-dimethyl-phenyl)acetyl]amino]-1,4-dioxaspiro[4.5]decan-8-carbonsäure-ropylester (79%) / 1-[[2-(4-Chlor-2,6-dimethyl-phenyl)acetyl]amino]-4,4-dipropoxy-cyclohexancarbonsäure-propylester (8%) / 1-[[2-(4-Chlor-2,6-dimethyl-phenyl)acetyl]amino]-4-oxo-cyclohexancarbonsäure-propylester (7%). Die Ausbeute beträgt 97%.

### Beispiel 18

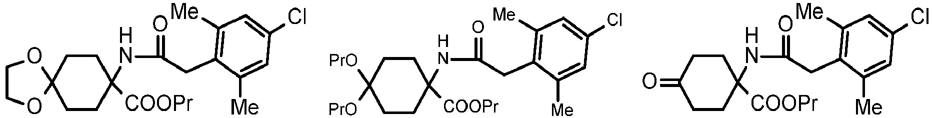

233.2g einer Lösung, die analog zu Beispiel 15 hergestellt wurde, wird bei 25-30°C Innentemperatur und 25mbar andestilliert. Nach Entfernung von 130.6g Destillat wird das Vakuum mit Stickstoff gebrochen und der Ansatz auf Normaldruck gebracht. Nach Abkühlen der Mischung auf 0-10°C werden 43.8g Na₂CO₃ gelöst in 381.7g Wasser sowie 32.6g 32%ige Natronlauge langsam zudosiert, so dass die Temperatur stets unter 5°C bleibt. Nach beendeter Dosierung werden nun 121.9g 2-(4-Chlor-2,6-dimethyl-phenyl)acetylchlorid (39.5%ig in Toluol) in einer Stunde addiert, so dass die Innentemperatur 5°C nicht übersteigt. Der Ansatz wird 30min bei 0-5°C nachgerührt und anschließend auf 72°C erwärmt. Die organische Phase wird abgetrennt und am Rotationsverdampfer eingeengt. Man erhält 97.1g eines blassgelben Feststoffes aus 8-[[2-(4-Chlor-2,6-dimethyl-phenyl)acetyl]amino]-1,4-dioxaspiro[4.5]decan-8-carbonsäure-propylester (78%) / 1-[[2-(4-Chlor-2,6-dimethyl-phenyl)acetyl]amino]-4,4-dipropoxy-cyclohexancarbonsäure-propylester (8%). Die Ausbeute beträgt 89%.

### Beispiel 19

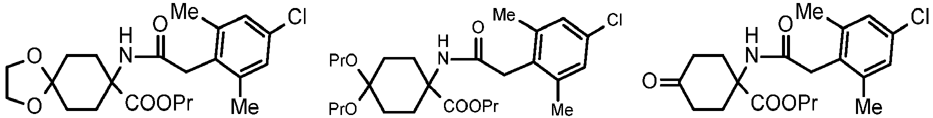

Von der in Beispiel 15 beschriebenen Lösung werden 227.8g vorgelegt und auf 40°C erwärmt, 400g Toluol addiert und bei Unterdruck bis zu 25mbar andestilliert. Nach Entfernung von 126.1g Destillat werden 400g Toluol addiert und bei 54-70mbar weitere 265.8g Destillat entfernt. Das Vakuum wird mit Stickstoff gebrochen und der Ansatz auf Normaldruck gebracht Es wird auf 0-5°C abgekühlt und eine Lösung von 41.7g Na₂CO₃ in 208g Wasser innerhalb von 40min zudosiert. Zu der erhaltenen weißen Suspension werden 128.5g 2-(4-Chlor-2,6-dimethyl-phenyl)acetylchlorid (39.9%ig in Toluol) bei 0-5°C innerhalb von 2h zudosiert. Die Mischung wird nach beendeter Dosierung auf 77.4°C erwärmt, die Phasen voneinander getrennt und die organische Phase am Rotationsverdampfer eingeengt. Man erhält 100.6g eines Gemisches aus 8-[[2-(4-Chloro-2,6-dimethyl-phenyl)acetyl]amino]-1,4-dioxaspiro[4.5]decan-8-carbonsäure-propylester (87%) / 1-[[2-(4-Chlor-2,6-dimethyl-phenyl)acetyl]amino]-4,4-dipropoxy-cyclohexancarbonsäure-propylester (1%) / 1-[[2-(4-Chlor-2,6-dimethyl-phenyl)acetyl]amino]-4-oxo-cyclohexancarbonsäure-propylester(9%). Die Ausbeute beträgt 99%.

### Beispiel 20

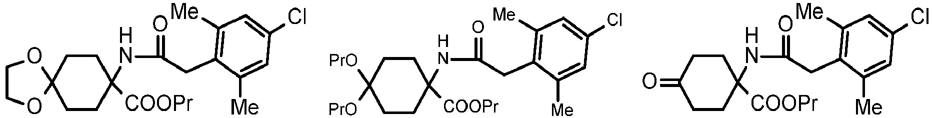

225.4g einer Lösung, die analog zu Beispiel 15 hergestellt wurde, wird bei 25-30°C Innentemperatur und 25mbar andestilliert. Nach Entfernung von 119.8g Destillat wird das Vakuum mit Stickstoff gebrochen und der Ansatz auf Normaldruck gebracht. 400g Toluol werden addiert und erneut bei 50-80mbar andestilliert. Während der Destillation wird fortlaufend Toluol nachdosiert. Auf diese Weise wurden weitere 279.9g Destillat erhalten und insgesamt 250g Toluol nachdosiert. Es wurde nun auf Normaldruck belüftet und auf 15-20°C abgekühlt. Bei dieser Temperatur werden zunächst 41.4g Na₂CO₃ in 208.6g Wasser in 30min zudosiert und im Anschluss 128.5g 2-(4-Chlor-2,6-dimethyl-phenyl)acetylchlorid (39.5% in Toluol) innerhalb von 90min zugetropft, so dass die Temperatur 20°C nicht übersteigt. Es wird 1h bei 20°C nachgerührt und anschließend auf 78°C erwärmt. Die organische Phase wird abgetrennt und am Rotationsverdampfer eingeengt. Man erhält 98.1g eines blassgelben Feststoffs aus 8-[[2-(4-Chloro-2,6-dimethyl-phenyl)acetyl]amino]-1,4-dioxaspiro[4.5]decan-8-carbonsäure-propylester (87%) / 1-[[2-(4-Chlor-2,6-dimethyl-phenyl)acetyl]amino]-4,4-dipropoxy-cyclohexancarbonsäure-propylester (2%) / 1-[[2-(4-Chlor-2,6-dimethyl-phenyl)acetyl]amino]-4-oxo-cyclohexancarbonsäure-propylester(9%). Die Ausbeute beträgt 98%.

### Beispiel 21

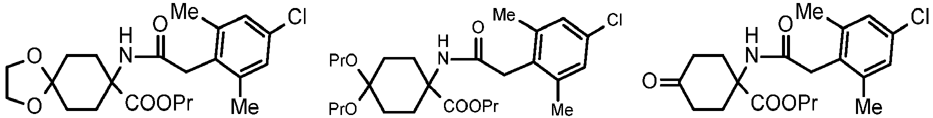

225.6g einer Lösung, die analog zu Beispiel 15 hergestellt wurde, wird bei 30-40°C Innentemperatur und 25mbar andestilliert. Nach Entfernung von 120.5g Destillat wird das Vakuum mit Stickstoff gebrochen und der Ansatz auf Normaldruck gebracht. 400g Toluol werden addiert und erneut bei 50-80mbar andestilliert. Während der Destillation wird fortlaufend Toluol nachdosiert. Auf diese Weise werden weitere 191.5g Destillat erhalten und insgesamt 189g Toluol nachdosiert. Es wird auf Normaldruck belüftet und auf 40°C erwärmt. Bei dieser Temperatur werden zunächst 41.4g Na₂CO₃ in 208.6g Wasser in 20min zudosiert und im Anschluss 128.16g 2-(4-Chlor-2,6-dimethyl-phenyl)acetylchlorid (39.5% in Toluol) innerhalb von 90min zugetropft, so dass die Temperatur 40°C nicht übersteigt. Es wird 15min bei 40°C nachgerührt und anschließend auf 77°C erwärmt. Die organische Phase wird abgetrennt und am Rotationsverdampfer eingeengt. Man erhält 101.92g eines blassgelben Feststoffs aus 8-[[2-(4-Chloro-2,6-dimethyl-phenyl)acetyl]amino]-1,4-dioxaspiro[4.5]decan-8-carbonsäure-propylester (88%) / 1-[[2-(4-Chlor-2,6-dimethyl-phenyl)acetyl]amino]-4,4-dipropoxy-cyclohexancarbonsäure-propylester (7%) / 1-[[2-(4-Chlor-2,6-dimethyl-phenyl)acetyl]amino]-4-oxo-cyclohexancarbonsäure-propylester (3%). Die Ausbeute beträgt 99%.

### Beispiel 22

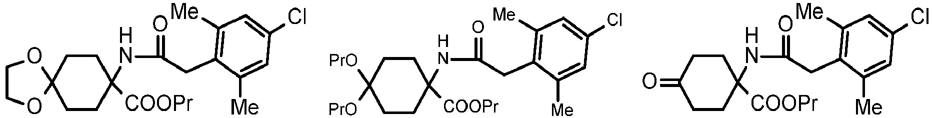

223.5g einer Lösung, die analog zu Beispiel 15 hergestellt wurde, wird bei 30-40°C Innentemperatur und 25mbar andestilliert. Nach Entfernung von 123.7g Destillat wird das Vakuum mit Stickstoff gebrochen und der Ansatz auf Normaldruck gebracht. 400g Toluol werden addiert und erneut bei 50-80mbar andestilliert. Während der Destillation wird fortlaufend Toluol nachdosiert. Auf diese Weise werden weitere 231.75g Destillat erhalten und insgesamt 200g Toluol nachdosiert. Es wurde nun auf Normaldruck belüftet und auf 40°C erwärmt. Bei dieser Temperatur werden zunächst 41.4g Na₂CO₃ in 208.6g Wasser in 20min zudosiert und im Anschluss 133.65g 2-(4-Chlor-2,6-dimethyl-phenyl)acetylchlorid (39.5% in Toluol) innerhalb von 90min zugetropft, so dass die Temperatur 40°C nicht übersteigt. Es wird 15min bei 40°C nachgerührt und anschließend auf 77°C erwärmt. Die organische Phase wird abgetrennt und am Rotationsverdampfer eingeengt. Man erhält 103.4g eines blassgelben Feststoffs aus 8-[[2-(4-Chloro-2,6-dimethyl-phenyl)acetyl]amino]-1,4-dioxaspiro[4.5]decan-8-carbonsäure-propylester (87%) / 1-[[2-(4-Chlor-2,6-dimethyl-phenyl)acetyl]amino]-4,4-dipropoxy-cyclohexancarbonsäure-propylester (1%) / 1-[[2-(4-Chlor-2,6-dimethyl-phenyl)acetyl]amino]-4-oxo-cyclohexancarbonsäure-propylester(8%). Die Ausbeute beträgt 98%.

### Beispiel 22a

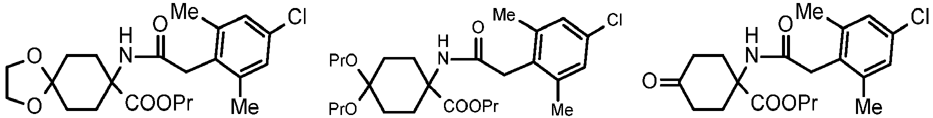

183.22g einer Lösung, die analog zu Beispiel 15 hergestellt wurde, wird bei 30-40°C Innentemperatur und 25mbar andestilliert. Nach Entfernung von 102,13g Destillat wird das Vakuum mit Stickstoff gebrochen und der Ansatz auf Normaldruck gebracht. 330g Toluol werden addiert, die Mischung auf 60°C erwärmt und erneut bei 100-300mbar andestilliert. Während der Destillation wird fortlaufend Toluol nachdosiert. Auf diese Weise werden weitere 127.5g Destillat erhalten und insgesamt 107,6g Toluol nachdosiert. Es wurde nun auf Normaldruck belüftet und bei 60°C zunächst 33.5g Na₂CO₃ in 171.3g Wasser in 20min zudosiert und im Anschluss 133.65g 2-(4-Chlor-2,6-dimethyl-phenyl)acetylchlorid (39.5% in Toluol) innerhalb von 90min zugetropft, so dass die Temperatur 60°C nicht übersteigt. Es wird 15min bei 40°C nachgerührt und anschließend auf 77°C erwärmt. Die organische Phase wird abgetrennt und am Rotationsverdampfer eingeengt. Man erhält 84.5g eines gelben Feststoffs aus 8-[[2-(4-Chloro-2,6-dimethyl-phenyl)acetyl]amino]-1,4-dioxaspiro[4.5]decan-8-carbonsäure-propylester (89%) / 1-[[2-(4-Chlor-2,6-dimethyl-phenyl)acetyl]amino]-4,4-dipropoxy-cyclohexancarbonsäure-propylester (0%) / 1 - [[2-(4-Chlor-2,6-dimethyl-phenyl)acetyl] amino] -4-oxo-cyclohexancarbonsäure-propylester(7 %). Ausbeute 97%.

### Beispiel 23

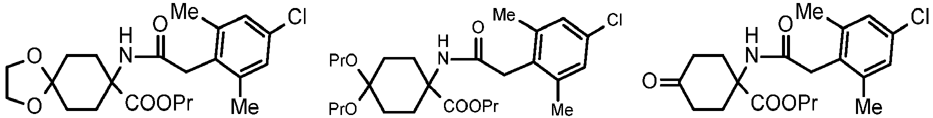

180.4g einer Lösung, die analog zu Beispiel 15 hergestellt wurde, wird bei 30-40°C Innentemperatur und 25mbar andestilliert. Nach Entfernung von 94.5g Destillat wird das Vakuum mit Stickstoff gebrochen und der Ansatz auf Normaldruck gebracht. 330g Toluol werden addiert, die Mischung auf 70-80°C erwärmt und erneut bei 300-400mbar andestilliert. Während der Destillation wird fortlaufend Toluol nachdosiert. Auf diese Weise werden weitere 100g Destillat erhalten und insgesamt 69.9g Toluol nachdosiert. Es wurde nun auf Normaldruck belüftet und bei 60-80°C zunächst 33.5g Na₂CO₃ in 171.3g Wasser in 20min zudosiert und im Anschluss 107.9g 2-(4-Chlor-2,6-dimethyl-phenyl)acetylchlorid (39.5% in Toluol) innerhalb von 90min zugetropft, so dass die Temperatur 80°C nicht übersteigt. Es wird 15min bei 80°C nachgerührt und anschließend auf 77°C erwärmt. Die organische Phase wird abgetrennt und am Rotationsverdampfer eingeengt. Man erhält 81.9g eines gelben Feststoffs aus 8-[[2-(4-Chloro-2,6-dimethyl-phenyl)acetyl]amino]-1,4-dioxaspiro[4.5]decan-8-carbonsäure-propylester (88%) / 1-[[2-(4-Chlor-2,6-dimethyl-phenyl)acetyl]amino]-4,4-dipropoxy-cyclohexancarbonsäure-propylester (0%) / 1-[[2-(4-Chlor-2,6-dimethyl-phenyl)acetyl]amino]-4-oxo-cyclohexancarbonsäure-propylester(6%). Die Ausbeute beträgt 93%.

### Beispiel 24

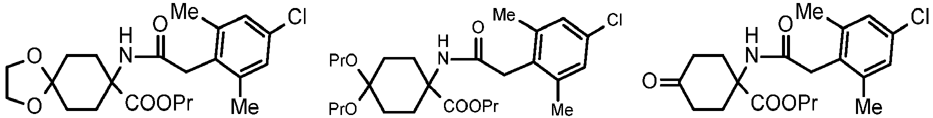

184.4g einer Lösung, die analog zu Beispiel 15 hergestellt wurde, wird bei 30-40°C Innentemperatur und 25mbar andestilliert. Nach Entfernung von 99.2g Destillat wird das Vakuum mit Stickstoff gebrochen und der Ansatz auf Normaldruck gebracht. 330g Chlorbenzol werden addiert, die Mischung auf 40°C erwärmt und erneut bis auf 35mbar andestilliert. Während der Destillation wird fortlaufend Chlorbenzol nachdosiert. Auf diese Weise werden weitere 193.2g Destillat erhalten und insgesamt 200g Chlorbenzol nachdosiert. Es wurde nun auf Normaldruck belüftet und bei 40°C zunächst 33.7g Na₂CO₃ in 171.3g Wasser in 20min zudosiert und im Anschluss 109.6g 2-(4-Chlor-2,6-dimethyl-phenyl)acetylchlorid (39.8% in Toluol) innerhalb von 90min zugetropft, so dass die Temperatur 40°C nicht übersteigt. Es wird 30min bei 40°C nachgerührt und anschließend auf 75°C erwärmt. Die organische Phase wird abgetrennt und am Rotationsverdampfer eingeengt. Man erhält 81.4g eines gelben Feststoffs aus 8-[[2-(4-Chloro-2,6-dimethyl-phenyl)acetyl]amino]-1,4-dioxaspiro[4.5]decan-8-carbonsäure-propylester (86%) / 1-[[2-(4-Chlor-2,6-dimethyl-phenyl)acetyl]amino]-4,4-dipropoxy-cyclohexancarbonsäure-propylester (3%) / 1-[[2-(4-Chlor-2,6-dimethyl-phenyl)acetyl]amino]-4-oxo-cyclohexancarbonsäure-propylester(5%). Die Ausbeute beträgt 90%.

### Beispiel 25

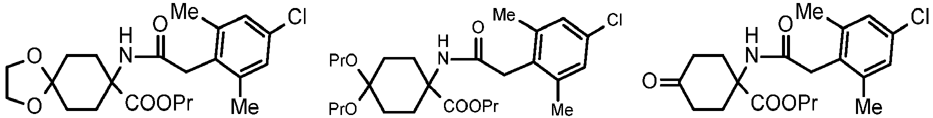

184.4g einer Lösung, die analog zu Beispiel 15 hergestellt wurde, wird bei 30-40°C Innentemperatur und 25mbar andestilliert. Nach Entfernung von 99.5g Destillat wird das Vakuum mit Stickstoff gebrochen und der Ansatz auf Normaldruck gebracht. 330g Chlorbenzol werden addiert, die Mischung auf 40°C erwärmt und erneut bis auf 35mbar andestilliert. Während der Destillation wird fortlaufend Chlorbenzol nachdosiert. Auf diese Weise werden weitere 122g Destillat erhalten und insgesamt 120g Chlorbenzol nachdosiert. Es wird auf Normaldruck belüftet und bei 40°C zunächst 33.7g Na₂CO₃ in 171.3g Wasser in 20min zudosiert und im Anschluss 111.9g 2-(4-Chlor-2,6-dimethyl-phenyl)acetylchlorid (39.1% in Chlorbenzol) innerhalb von 90min zugetropft, so dass die Temperatur 40°C nicht übersteigt. Es wird 30min bei 40°C nachgerührt und anschließend auf 75°C erwärmt. Die organische Phase wird abgetrennt und am Rotationsverdampfer eingeengt. Man erhält 86.7g eines gelben Feststoffs aus 8-[[2-(4-Chloro-2,6-dimethyl-phenyl)acetyl]amino]-1,4-dioxaspiro[4.5]decan-8-carbonsäure-propylester (82%) / 1-[[2-(4-Chlor-2,6-dimethyl-phenyl)acetyl]amino]-4,4-dipropoxy-cyclohexancarbonsäure-propylester (7%) / 1-[[2-(4-Chlor-2,6-dimethyl-phenyl)acetyl]amino]-4-oxo-cyclohexancarbonsäure-propylester(4%). Die Ausbeute beträgt 95%.

### Beispiel 26

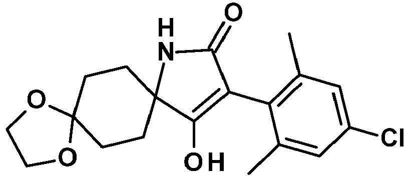

60g 8-Amino-1,4-dioxaspiro[4.5]decan-8-carbonsäure werden in 196g 1-Propanol vorgelegt und auf 70-80°C erwärmt. Zu der Mischung werden 39g Thionylchlorid innerhalb von 30min zugetropft. Entstehendes Gas wird über einen Natronlaugenwäscher abgeführt. Der Ansatz wird bei 80-90°C für 4h nachgerührt. Die Mischung wird auf 45°C abgekühlt und im Vakuum andestilliert. Man erhält 120g Destillat. Zu dem Destillationssumpf werden 455g Toluol gegeben und weitere 164g Destillat abgenommen. Der Ansatz wird auf -5 bis 5°C abgekühlt und 50g Natriumcarbonat gelöst in 274g Wasser werden in 40min zudosiert. Im Anschluss werden 152.8g 2-(4-Chlor-2,6-dimethyl-phenyl)acetylchlorid (40% in Toluol) zudosiert so dass 5°C nicht überschritten werden. Die enstandenen Suspension wird auf 70-80°C erwärmt, die untere wässrige Phase wird abgetrennt, die obere organische Phase wird einmal mit 70g Wasser bei 80°C gewaschen. Zu der organischen Phase werden 9g Ethandiol sowie 2.8g 7%ige Salzsäure gegeben und aus der Mischung bei 102-110°C Wasser ausgekreist. Nach 3h wird das Toluol abdestilliert, der Sumpf in 123g DMAc aufgenommen und nochmals im Vakuum bei etwa 100°C andestilliert. Zu der verbliebenen Reaktionsmischung werden 101.4g einer 30%igen Natriummethanolatlösung innerhalb einer Stunde addiert. Bei leichtem Unterdruck wird kontinuierlich anfallendes Methanol und Propanol abdestilliert. Der Ansatz wird 4h nachgerührt, danach werden 352g Wasser addiert und 1h bei 95°C nachgerührt. Die Lösung wird auf 80°C abgekühlt und 56g 37%iger Salzsäure zudosiert. Es wird innerhalb von 2h auf Raumtemperatur abgekühlt, die entstandene Suspension wird filtriert, der Filterkuchen mit 2x120g Wasser gewaschen und im Vakuum bei 50°C getrocknet. Man erhält 96g 2-(4-Chlor-2,6-dimethyl-phenyl)-1-hydroxy-9,12-dioxa-4-azadispiro[4.2.4⁸.2⁵]tetradec-1-en-3-on mit einer Reinheit von 94%, Die Ausbeute beträgt 88%.

### Beispiel 27

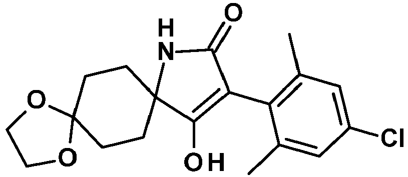

100g 8-Amino-1,4-dioxaspiro[4.5]decan-8-carbonsäure werden in 319g 1-Propanol vorgelegt. Bei 70-85°C werden 63.2g Thionylchlorid in einer Stunde zudosiert und dann 1h bei 80-90°C nachgerührt. Der Ansatz wird auf 30°C gekühlt und im Vakuum bei 30-50mbar andestilliert. Man erhält 130g Destillat. Es werden 470g Toluol nachgesetzt und durch erneutes Andestillieren weitere 333g Destillat abgenommen. Der Sumpf wird auf -5 bis 5°C gekühlt, 87g Na₂CO₃ in 435g Wasser geloest in einer Stunde zudosiert und danach 265g 2-(4-Chlor-2,6-dimethyl-phenyl)acetylchlorid als 39.6%ige Lösung in Toluol innerhalb von 40min zudosiert. Es wird 1h bei Raumtemperatur nachgerührt und auf 80°C erwärmt. Nach Abtrennen der unteren wässrigen Phase wird die verbliebene organische Phase mit 50g Wasser gewaschen. Im Anschluss werden 9.2g para-Toluolsulfonsäure und 30g Ethandiol zugesetzt, auf Rückfluss erwärmt und Wasser über 3h ausgekreist. Das Reaktionsgemisch wird danach andestilliert (308g Destillat) und100g DMAc zugegeben. Die Mischung wird erneut andestilliert und 15g Destillat werden abgenommen. Zu der verbliebenen Mischung werden135g Natriummethylat (30%ige Lösung in Methanol) bei 110°C in 30min zudosiert und über 3h unter leichtem Vakuum anfallendes Methanol und 1-Propanol abdestilliert. Es werden 235g Wasser zugegeben und die Mischung bei 90-100°C über 1h gerührt. Danach wird auf 80°C gekühlt, dann 46.4g Essigsäure in 30min addiert, in 3h auf Raumtemperatur abgekühlt und die entstandene Suspension abfiltriert. Der Filterkuchen wird mit 2x150g Wasser gewaschen. Man erhält 165g 2-(4-Chlor-2,6-dimethyl-phenyl)-1-hydroxy-9,12-dioxa-4-azadispiro[4.2.4⁸.2⁵]tetradec-1-en-3-on mit einer Reinheit von 87%, Die Ausbeute beträgt 82%.

### Beispiel 28

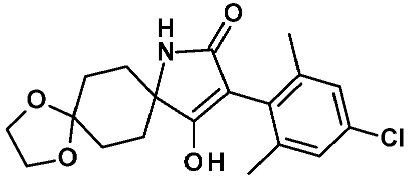

109g 8-Amino-1,4-dioxaspiro[4.5]decan-8-carbonsäure(96.9%ig) werden in 357g 1-Propanol vorgelegt. Bei 70-80°C werden 71g Thionylchlorid in 50min zudosiert und der Ansatz nach beendeter Dosierung für etwa 4h nachgerührt. Es wird bei maximal 45°C Mantel und Vakuum bis 23mbar andestilliert. Nach Entfernung von 178g Destillat werden 830g Toluol addiert, weitere 462g Destillat abgenommen und auf -5 bis 5°C abgekühlt. 91g Na₂CO₃ in 500g Wasser werden in einer Stunde addiert, gefolgt von 280g 2-(4-Chlor-2,6-dimethyl-phenyl)acetylchlorid (40% in Toluol) ebenfalls in 1h dosiert. Die Mischung wird auf 80-90°C erwärmt, die wässrige Phase abgetrennt und die organische Phase mit 9g para-Toluolsulfonsäure sowie 32g Ethandiol versetzt. Mit aufgesetztem Waserabscheider wird bei Normaldruck 5h refluxiert. Das Toluol wird abdestilliert und der verbliebene Sumpf in 224g DMAc aufgenommen. Die Mischung wird leicht andestilliert und bei 100-120°C 185g Natriummethanolat (30%ige Lösung in Methanol) zudosiert. Methanol und 1-Propanol werden über 2h unter leichtem Vakuum bei 100-120°C abdestilliert. Zu dem Ansatz werden 600g Wasser addiert und 1h bei 85-100°C nachgerührt. Es wird auf 80°C abgekühlt und 97g Essigsäure in 30min addiert. Danach wird auf Raumtemperatur abgekühlt, die Suspension abgesaugt und der Filterkuchen mit 2x 180g Wasser gewaschen. Man erhält nach Trocknen im Vakuum 171.5g 2-(4-Chlor-2,6-dimethyl-phenyl)-1-hydroxy-9,12-dioxa-4-azadispiro[4.2.4⁸.2⁵]tetradec-1-en-3-on mit 94% Reinheit und 86% Ausbeute.

### Beispiel 29

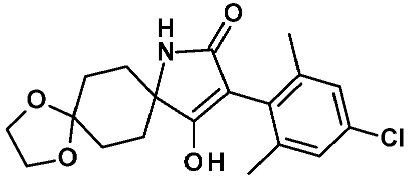

60g 8-Amino-1,4-dioxaspiro[4.5]decan-8-carbonsäure werden in 130g 1-Propanol und 140g Chlorbenzol vorgelegt und auf 70-80°C erwärmt. Zu der Mischung werden 70g Thionylchlorid innerhalb von 30min zugetropft. Entstehendes Gas wird über einen Natronlaugenwäscher abgeführt. Der Ansatz wird bei 80-90°C für 12h nachgerührt. Die Mischung wird auf 45°C abgekühlt und im Vakuum andestilliert. Man erhält 160g Destillat. Es werden 50g Natriumcarbonat, gelöst in 274g Wasser, in 40min zudosiert. Im Anschluss werden 152.8g 2-(4-Chlor-2,6-dimethyl-phenyl)acetylchlorid (40% in Toluol) zudosiert. Die enstandenene Suspension wird auf 70-80°C erwärmt, die untere wässrige Phase wird abgetrennt, die obere organische Phase wird einmal mit 150g Wasser bei 80°C gewaschen. Zu der organischen Phase werden 9g Ethandiol sowie 2.8g para-Toluolsulfonsäure gegeben und aus der Mischung bei 100-110°C unter leichtem Vakuum Wasser ausgekreist. Nach 2h werden 9g Ethandiol nachgesetzt und weitere 4h Wasser ausgekreist. Danach werden bei 110°C 105g einer 30%igen Natriummethanolatlösung innerhalb einer Stunde addiert. Bei leichtem Unterdruck wird kontinuierlich anfallendes Methanol und Propanol abdestilliert. Der Ansatz wird 4h nachgerührt, danach werden 325g Wasser addiert und 1h bei 95°C nachgerührt. Die Lösung wird auf 80°C abgekühlt und 43g Essigsäure zudosiert. Es wird innerhalb mehrerer Stunden auf Raumtemperatur abgekühlt, die entstandene Suspension wird filtriert, der Filterkuchen mit 2x60g Wasser gewaschen und im Vakuum bei 50°C getrocknet. Man erhält 87g 2-(4-Chlor-2,6-dimethyl-phenyl)-1-hydroxy-9,12-dioxa-4-azadispiro[4.2.4⁸.2⁵]tetradec-1-en-3-on mit einer Reinheit von 89%, Die Ausbeute beträgt 77%.

Die folgenden Beispiele starten von dem Produktgemisch 8-[[2-(4-Chlor-2,6-dimethylphenyl)acetyl]amino]-1,4-dioxaspiro[4.5]decan-8-carbonsäure-propylester / 1-[[2-(4-Chlor-2,6-dimethyl-phenyl)acetyl]amino]-4,4-dipropoxy-cyclohexancarbonsäure-propylester / 1-[[2-(4-Chlor-2,6-dimethyl-phenyl)acetyl]amino]-4-oxo-cyclohexancarbonsäure-propylester, können aber auch wie die Beispiele 26 bis 29 ohne jegliche Zwischenisolation beginnend von 8-Amino-1,4-dioxaspiro[4.5]decan-8-carbonsäure oder dessen entsprechenden Natrium- bzw. Kalium-Salzen ebenfalls durchgeführt werden.

### Beispiel 30

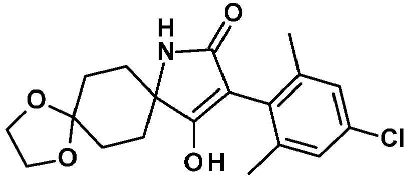

50g eines Gemischs hergestellt analog zu Beispielen 15-25 in 116.7g Chlorbenzol werden mit 1.5g 37%ige Salzsäure und 3.8g Ethandiol versetzt, auf Reflux erhitzt, nach 4h mit weiteren 1.5g Salzsäure versetzt und für weitere 3h bei Reflux gerührt. Es wird auf 115°C abgekühlt und 42.8g Natriummethanolat (30%ige Lösung in Methanol) bei leichtem Unterdruck in 30min zudosiert. Es wird fortlaufend über 2h anfallendes Methanol und 1-Propanol abdestilliert. Zu der Mischung werden 118g Wasser gegeben und bei 90-100°C für 1h gerührt. Der Ansatz wird auf 80°C gekühlt und 23.2g 37%ige Salzsäure in 30min zudosiert. Die Mischung wird über mehrere Stunden auf Raumtemperatur abgekühlt, die entstandene Suspension nochmals mit 50g Wasser versetzt, um die Rührbarkeit zu erhalten und dann filtriert. Der Filterkuchen wird mit 3x 60g Wasser gewaschen. Man erhält 33.9g 2-(4-Chlor-2,6-dimethyl-phenyl)-1-hydroxy-9,12-dioxa-4-azadispiro[4.2.4⁸.2⁵]tetradec-1-en-3-on mit einer Reinheit von 95%, Die Ausbeute beträgt 87% bezogen auf das eingesetzte Gemisch.

### Beispiel 31

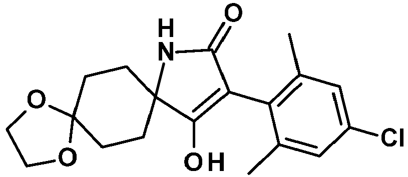

50g eines Gemischs hergestellt analog zu Beispielen 15-25 in 116.7g Chlorbenzol werden mit 1.5g 37%ige Salzsäure und 3.8g Ethandiol versetzt und mit aufgesetztem Wasserabscheider für 3h bei Reflux gerührt. Das Chlorbenzol wird leicht andestilliert, mit 100g DMAc versetzt und das Chlorbenzol bei 130°C Manteltemperatur und Vakuum abdestilliert. Zu der Mischung wird bei 94°C 42.8g Natriummethanolat in 30min zudosiert und über 2h unter leichtem Vakuum 1-Propanol und Methanol abdestilliert. Zu der Mischung werden dann 118g Wasser zugegeben und 1h bei 90-100°C nachgerührt. Es wird auf 80°C gekühlt und 23.2g 37%ige Salzsäure in 30min addiert. Die Mischung wird auf 10°C abgekühlt und filtriert. Der Filterkuchen wird mit 60g Wasser gewaschen und im Vakuum bei 50°C getrocknet. Man erhält 39.2g 2-(4-Chlor-2,6-dimethyl-phenyl)-1-hydroxy-9,12-dioxa-4-azadispiro[4.2.4⁸.2⁵]tetradec-1-en-3-on mit einer Reinheit von 90%, Die Ausbeute beträgt 95%, bezogen auf das eingesetzte Gemisch.

### Beispiel 32

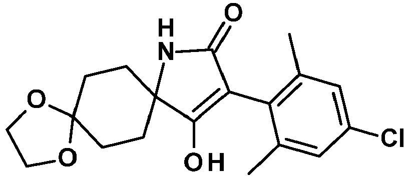

50g eines Gemischs hergestellt analog zu Beispielen 15-25 in 116.7g Anisol werden mit 5.3g para-Toluolsulfonsäure und 6.5g Ethandiol versetzt und für 3h bei Reflux gerührt. Es wird auf 115°C abgekühlt und 42.8g Natriummethanolat (30%ige Lösung in Methanol) bei leichtem Unterdruck in 30min zudosiert. Es wird fortlaufend über 2h anfallendes Methanol und 1-Propanol abdestilliert. Zu der Mischung werden 118g Wasser gegeben und bei 90-100°C für 1h gerührt. Der Ansatz wird auf 80°C gekühlt und 20.8g Essigsäure in 30min zudosiert. Die Mischung wird über mehrere Stunden auf Raumtemperatur abgekühlt und die entstandene Suspension filtriert. Der Filterkuchen wird mit 2x 60g Wasser gewaschen. Man erhält 35g 2-(4-Chlor-2,6-dimethyl-phenyl)-1-hydroxy-9,12-dioxa-4-azadispiro[4.2.4⁸.2⁵]tetradec-1-en-3-on mit einer Reinheit von 87%, Die Ausbeute beträgt 80%, bezogen auf das eingesetzte Gemisch.

### Beispiel 33

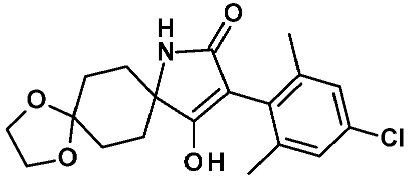

40g eines Gemischs hergestellt analog zu Beispielen 15-25 in 115g Toluol werden mit 1.5g para-Toluolsulfonsäure und 5.4g Ethandiol versetzt, auf Reflux erhitzt, nach 2h wird der Großteil des Toluols abdestilliert und der Sumpf mit 40g DMAc versetzt. Es werden ~8g Destillat aus der Mischung abgenommen , auf 115°C abgekühlt und 28.1g Natriummethanolat (30%ige Lösung in Methanol) bei leichtem Unterdruck in 30min zudosiert. Es wird fortlaufend über 3h anfallendes Methanol und 1-Propanol abdestilliert. Zu der Mischung werden 95g Wasser gegeben und bei 90-100°C für 1h gerührt. Der Ansatz wird auf 80°C gekühlt und 19g Essigsäure in 30min zudosiert. Die Mischung wird über 3h auf 10°C abgekühlt, die entstandene Suspension dann filtriert. Der Filterkuchen wird mit 2x50g Wasser gewaschen. Man erhält 31.5g 2-(4-Chlor-2,6-dimethyl-phenyl)-1-hydroxy-9,12-dioxa-4-azadispiro[4.2.4⁸.2⁵]tetradec-1-en-3-on mit einer Reinheit von 97%, Die Ausbeute beträgt 91%, bezogen auf das eingesetzte Gemisch.

### Beispiel 34

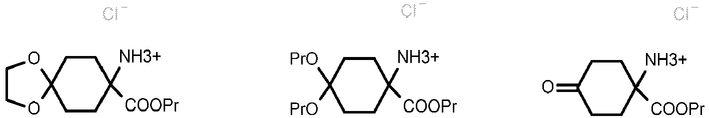

50g 8-Amino-1,4-dioxaspiro[4.5]decan-8-carbonsäure werden in 59g 1-Propanol und 100g Toluol vorgelegt, auf 80°C erwärmt und zu der Mischung 29g Thionylchlorid in einer Stunde zudosiert. Nach 5h zeigt die Umsatzkontrolle noch 20% Edukt an.

### Beispiel 35

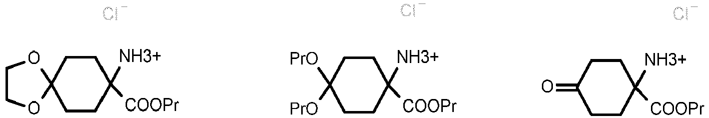

60g 8-Amino-1,4-dioxaspiro[4.5]decan-8-carbonsäure werden in 86g 1-Propanol und 120g Chlorbenzol vorgelegt, auf 80°C erwärmt und von der Mischung eine Probe gezogen: Die Umsatzkontrolle zeigt ~ 25% Gehalt an Edukt an. Zu der Mischung werden 39g Thionylchlorid in einer Stunde zudosiert. Nach 5h bei 80-90°C zeigt die Umsatzkontrolle ~11%w/w Edukt. Es werden weitere 15g Thionylchlorid addiert und über 5h bei 80-90°C nachgerührt. Die erneute Umsatzkontrolle zeigt 3.8%w/w Edukt. Es werden nochmals 0.5eq Thionylchlorid addiert und weitere 4h bei 80-90°C gerührt. Die Umsatzkontrolle zeigt 0.3%w/w Edukt im Reaktionsgemisch an.

### Beispiel 36

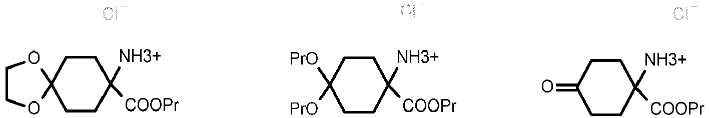

51g 8-Amino-1,4-dioxaspiro[4.5]decan-8-carbonsäure werden in 101g 1-Propanol vorgelegt, auf 80°C erwärmt und von der Mischung eine Probe gezogen: Umsatzkontrolle zeigt 23%w/w Gehalt an Edukt. Zu der Mischung werden 28.9g Thionylchlorid in einer Stunde zudosiert. Nach 5h bei 80-90°C zeigt die Umsatzkontrolle 1.4% Edukt im Reaktionsgemisch an.

### Beispiel 37

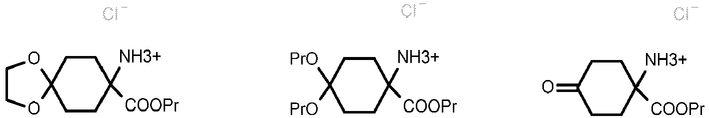

50g Natrium-8-amino-1,4-dioxaspiro[4.5]decan-8-carboxylat werden in 130g 1-Propanol vorgelegt, auf 80-90°C erwärmt und von der Mischung eine Probe gezogen: Umsatzkontrolle zeigt ~ 23%w/w Edukt. Zu der Mischung werden 46.9g Thionylchlorid in einer Stunde zudosiert. Nach 5h bei 80-90°C zeigt die Umsatzkontrolle 1.5% Edukt im Reaktionsgemisch an.

### Beispiel 38

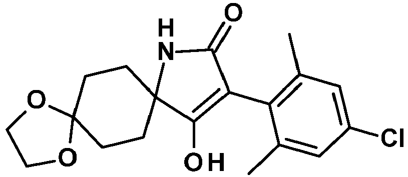

50g eines Gemischs (etwa 0.081 mmol) hergestellt analog zu Beispielen 15-25 in 94g Toluol werden mit 1g 96%iger Schwefelsäure und 5.9g Ethandiol versetzt und mit aufgesetztem Wasserabscheider, auf Reflux erhitzt. Nach 5h wird der Großteil des Toluol abdestilliert und 50g DMAc addiert. Nach erneutem Andestillieren der Mischung werden bei 115°C 34g Natriummethanolat (30%ige Lösung in Methanol) bei leichtem Unterdruck in 30min zudosiert. Es wird fortlaufend über 2h anfallendes Methanol und 1-Propanol abdestilliert. Zu der Mischung werden 95g Wasser gegeben und bei 90-100°C für 1h gerührt. Der Ansatz wird auf 80°C gekühlt und 11.3g Essigäure in 30min zudosiert. Die Mischung wird über mehrere Stunden auf Raumtemperatur abgekühlt, die entstandene Suspension dann filtriert. Der Filterkuchen wird mit 2x 50g Wasser gewaschen. Man erhält 28.5g 2-(4-Chlor-2,6-dimethyl-phenyl)-1-hydroxy-9,12-dioxa-4-azadispiro[4.2.48.25]tetradec-1-en-3-on mit einer Reinheit von 94%, Die Ausbeute beträgt 90% bezogen auf das eingesetzte Gemisch.

### Die Bezeichnung "Pr" in den Formelbildern steht für n-Propyl.

Die Bezeichnung "Bu" in den Formelbildern steht für n-Butyl.

## Patentansprüche

1. Verfahren zur Herstellung von Verbindungen der Formel (XI), **dadurch gekennzeichnet, dass** man Verbindungen der Formel (III) durch Umsetzung mit Verbindungen der Formel (XV)
R⁷-OH (XV),
und Thionylchlorid zu Gemischen der Hydrochloride der Verbindungen der Formeln (IV'), (V') und (VI') umsetzt, diese Hydrochloride mittels Basen in die freien Verbindungen der Formeln (IV`), (V`) und (VI') umsetzt, diese dann in Gegenwart einer Base mit Verbindungen der Formel (VII) acyliert, wobei Gemische der Verbindungen der Formeln (VIII'), (IX') und (X') erhalten werden, diese Verbindungen anschließend in einer Dieckmann-Reaktion durch Einwirkung einer starken Base zu Gemischen der Verbindungen der Formeln (XI), (XII') und (XIII) cyclisiert; diese Verbindungen dann in Gegenwart einer Säure mit Verbindungen der Formel (XIV)
HO-(CR¹R²)ₙ-CR³R⁴-CR⁵R⁶-OH (XIV)
zu Verbindungen der Formel (XI)
umsetzt: 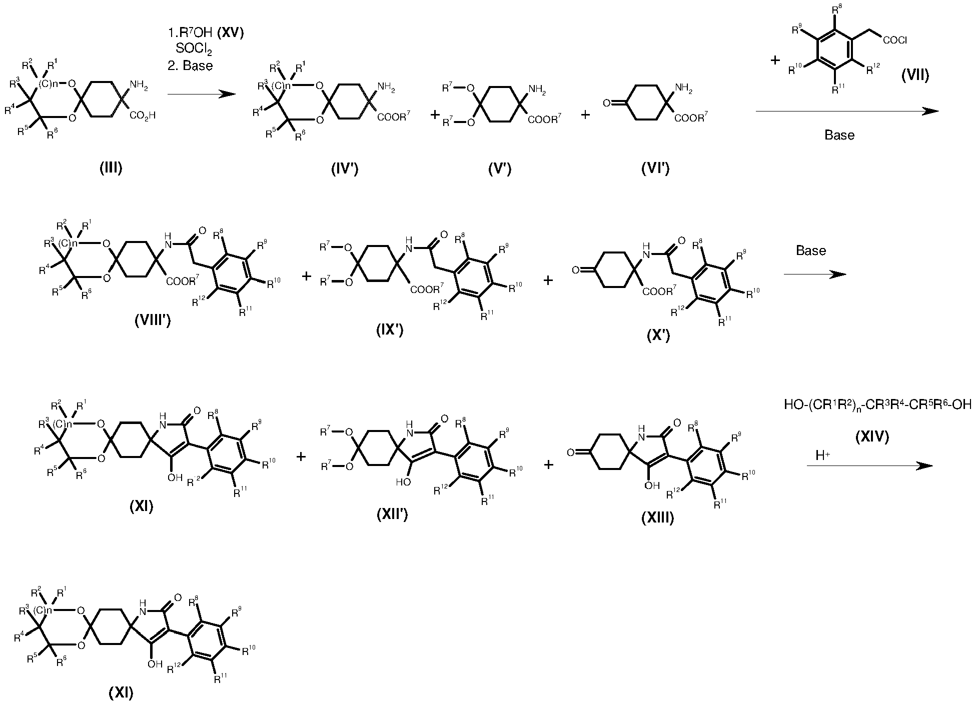 wobei
R³ für Wasserstoff steht,
R⁴ für Wasserstoff steht,
R⁵ für Wasserstoff steht,
R⁶ für Wasserstoff steht,
R⁷ für n-Propyl steht,
R⁸ für Methyl steht,
R⁹ für Wasserstoff steht,
R¹⁰ für Chlor steht,
R¹¹ für Wasserstoff steht
R¹² für Methyl steht,
n für 0 steht,
da n für 0 steht, sind die Substituenten R¹ und R² nicht definiert

2. Verbindungen der Formel (V') 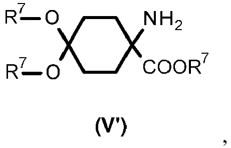 in welcher
R⁷ die in Anspruch 1 angegebene Bedeutung hat.

3. Verbindungen der Formel (IX') 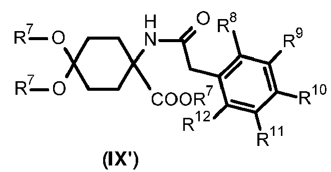 in welcher R⁷, R⁸, R⁹, R¹⁰, R¹¹, R¹² die oben angegebenen Bedeutungen haben.

4. Verbindungen der Formel (X') 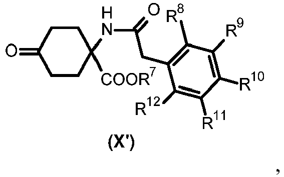 in welcher R⁷, R⁸, R⁹, R¹⁰, R¹¹, R¹² die oben angegebenen Bedeutungen haben.

5. Verbindungen der Formel (XII') 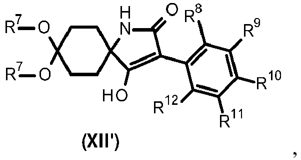 in welcher R⁷, R⁸, R⁹, R¹⁰, R¹¹, R¹² die oben angegebenen Bedeutungen haben.

6. Verbindungen der Formel (VI') 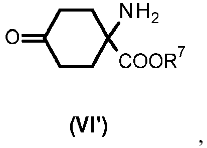 in welcher
R⁷ die in Anspruch 1 angegebene Bedeutung hat.

7. Verfahren zur Herstellung von Verbindungen der Formel (XI), **dadurch gekennzeichnet, dass** man Verbindungen Formel (III) durch Umsetzung mit Verbindungen der Formel (XV)
R⁷-OH (XV),
und Thionylchlorid zu Gemischen der Hydrochloride der Verbindungen der Formeln (IV'), (V') und (VI') umsetzt, diese Hydrochloride mittels Basen in die freien Verbindungen der Formeln (IV`), (V`) und (VI') umsetzt, diese dann in Gegenwart einer Base mit Verbindungen der Formel (VII) acyliert, wobei Gemische der Verbindungen der Formeln (VIII'), (IX') und (X') erhalten werden, diese Verbindungen dann in Gegenwart einer Säure mit Verbindungen der Formel (XIV)
HO-(CR¹R²)ₙ-CR³R⁴-CR⁵R⁶-OH (XIV)
zu Verbindungen der Formel (XVI)
umsetzt und diese Verbindungen anschließend in einer Dieckmann-Reaktion durch Einwirkung einer starken Base zu Verbindungen der Formeln (XI) cyclisiert: 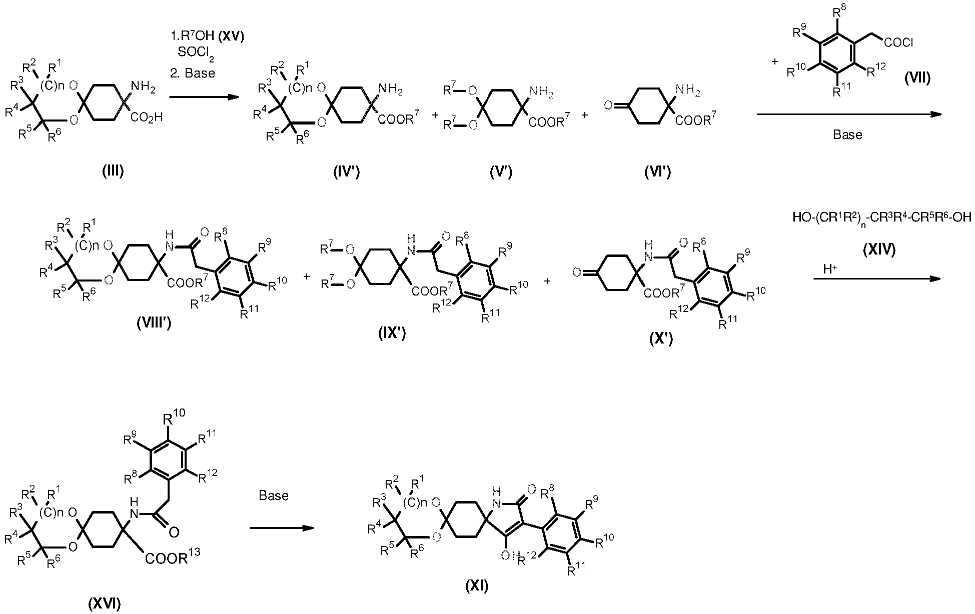 wobei
R³ für Wasserstoff steht,
R⁴ für Wasserstoff steht,
R⁵ für Wasserstoff steht,
R⁶ für Wasserstoff steht,
R⁷ für n-Propyl steht,
R⁸ für Methyl steht,
R⁹ für Wasserstoff steht,
R¹⁰ für Chlor steht,
R¹¹ für Wasserstoff steht,
R¹² für Methyl steht,
R¹³ für n-Propyl oder -CH₂CH₂-OH steht,
n für 0 steht,
da n für 0 steht, sind die Substituenten R¹ und R² nicht definiert.

## Claims

1. Process for preparing compounds of the formula (XI), **characterized in that** compounds of the formula (III) are converted by reaction with compounds of the formula (XV)
R⁷-OH (XV)
and thionyl chloride to give mixtures of the hydrochlorides of the compounds of the formulae (IV'), (V') and (VI'), these hydrochlorides are converted by means of bases to the free compounds of the formulae (IV'), (V') and (VI'), then these are acylated in the presence of a base with compounds of the formula (VII) to obtain mixtures of the compounds of the formulae (VIII'), (IX') and (X'), these compounds are then cyclized in a Dieckmann reaction by the action of a strong base to give mixtures of the compounds of the formulae (XI), (XII') and (XIII), then these compounds are reacted in the presence of an acid with compounds of the formula (XIV)
HO- (CR¹R²)ₙ-CR³R⁴-CR⁵R⁶-OH (XIV)
to give compounds of the formula (XI): 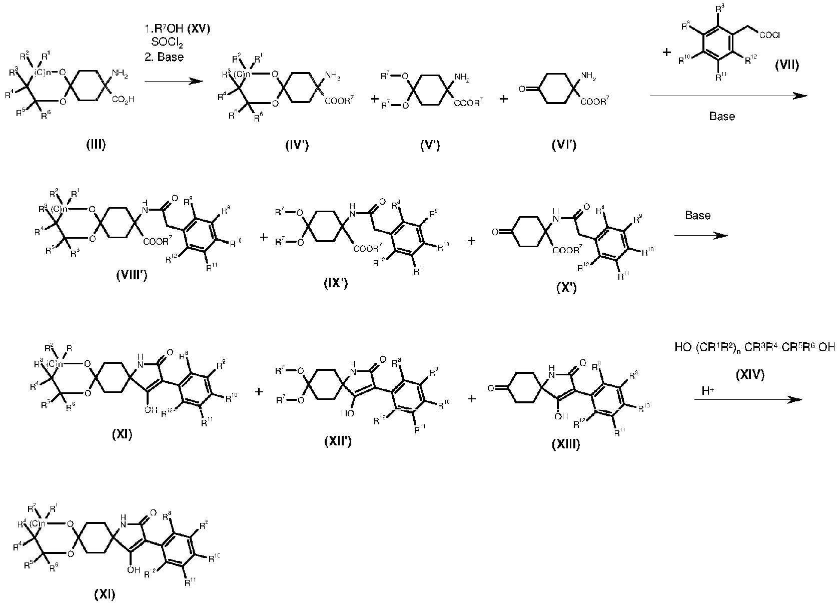 where
R³ is hydrogen,
R⁴ is hydrogen,
R⁵ is hydrogen,
R⁶ is hydrogen,
R⁷ is n-propyl,
R⁸ is methyl,
R⁹ is hydrogen,
R¹⁰ is chlorine,
R¹¹ is hydrogen,
R¹² is methyl,
n is 0;
since n is 0, the substituents R¹ and R² are undefined.

2. Compounds of the formula (V') 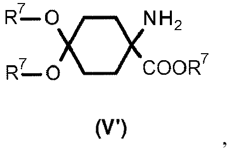 in which
R⁷ has the definition given in Claim 1.

3. Compounds of the formula (IX') 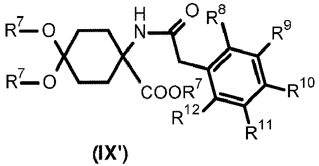 in which R⁷, R⁸, R⁹, R¹⁰, R¹¹, R¹² have the definitions given above.

4. Compounds of the formula (X') 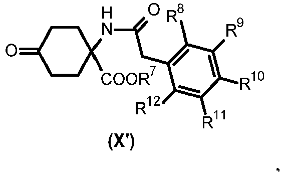 in which R⁷, R⁸, R⁹, R¹⁰, R¹¹, R¹² have the definitions given above.

5. Compounds of the formula (XII') 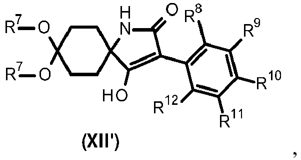 in which R⁷, R⁸, R⁹, R¹⁰, R¹¹, R¹² have the definitions given above.

6. Compounds of the formula (VI') 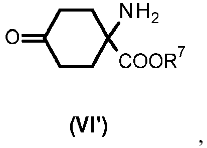 in which
R⁷ has the definition given in Claim 1.

7. Process for preparing compounds of the formula (XI), **characterized in that** compounds of the formula (III) are converted by reaction with compounds of the formula (XV)
R⁷-OH (XV)
and thionyl chloride to give mixtures of the hydrochlorides of the compounds of the formulae (IV'), (V') and (VI'), these hydrochlorides are converted by means of bases to the free compounds of the formulae (IV'), (V') and (VI'), then these are acylated in the presence of a base with compounds of the formula (VII) to obtain mixtures of the compounds of the formulae (VIII'), (IX') and (X'), then these compounds are reacted in the presence of an acid with compounds of the formula (XIV)
HO- (CR¹R²)ₙ-CR³R⁴-CR⁵R⁶-OH (XIV)
to give compounds of the formula (XVI)
and these compounds are then cyclized in a Dieckmann reaction by the action of a strong base to give compounds of the formula (XI) : 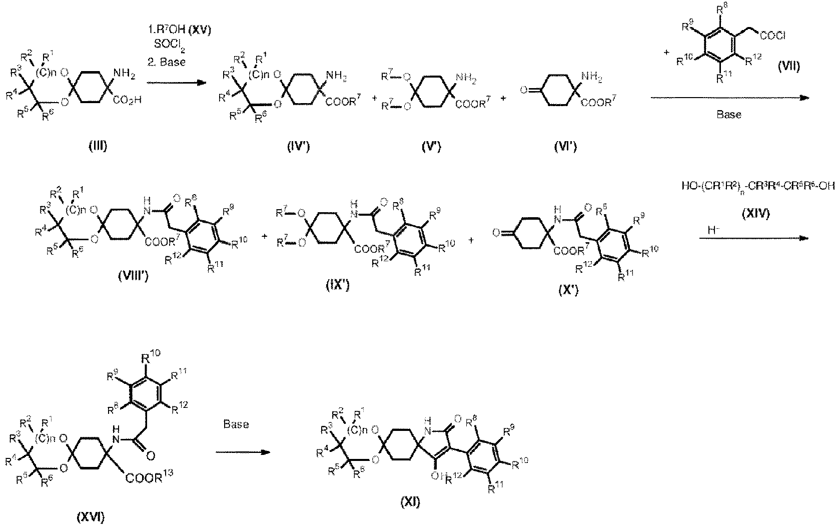 where
R³ is hydrogen,
R⁴ is hydrogen,
R⁵ is hydrogen,
R⁶ is hydrogen,
R⁷ is n-propyl,
R⁸ is methyl,
R⁹ is hydrogen,
R¹⁰ is chlorine,
R¹¹ is hydrogen,
R¹² is methyl,
R¹³ is n-propyl or -CH₂CH₂-OH,
n is 0;
since n is 0, the substituents R¹ and R² are undefined.

## Revendications

1. Procédé de préparation de composés de formule (XI), **caractérisé en ce qu'**on transforme des composés de formule (III) par réaction avec des composés de formule (XV)
R⁷-OH (XV),
et du chlorure de thionyle en mélanges des chlorhydrates des composés des formules (IV'), (V') et (VI'), on transforme ces chlorhydrates au moyen de bases en composés libres des formules (IV'), (V') et (VI'), on acyle ensuite ceux-ci en présence d'une base avec des composés de formule (VII), des mélanges des composés des formules (VIII'), (IX') et (X') étant obtenus, on cyclise ensuite ces composés dans une réaction de Dieckmann sous l'effet d'une base forte en mélanges des composés des formules (XI), (XII') et (XIII) ; on transforme alors ces composés en présence d'un acide avec des composés de formule (XIV)
HO- (CR¹R²)ₙ-CR³R⁴-CR⁵R⁶-OH (XIV)
en composés de formule (XI) 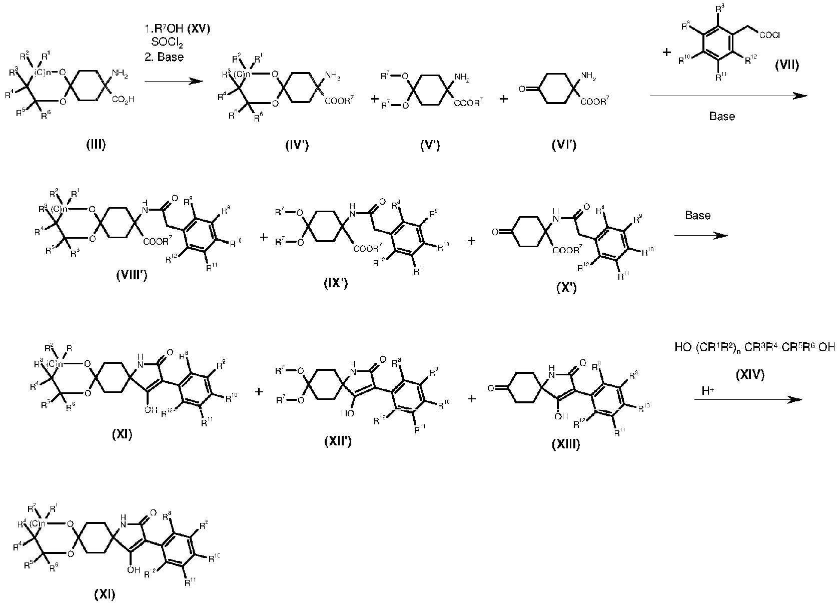 où
R³ représente hydrogène,
R⁴ représente hydrogène,
R⁵ représente hydrogène,
R⁶ représente hydrogène,
R⁷ représente n-propyle,
R⁸ représente méthyle,
R⁹ représente hydrogène,
R¹⁰ représente chlore,
R¹¹ représente hydrogène,
R¹² représente méthyle,
n vaut 0,
vu que n vaut 0, les substituants R¹ et R² ne sont pas définis.

2. Composés de formule (V') 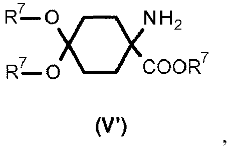 dans laquelle
R⁷ présente la signification indiquée dans la revendication 1.

3. Composés de formule (IX') 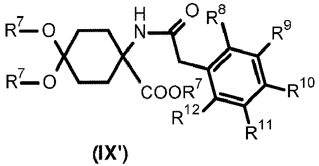 dans laquelle R⁷, R⁸, R⁹, R¹⁰, R¹¹, R¹² présentent les significations indiquées ci-dessus.

4. Composés de formule (X') 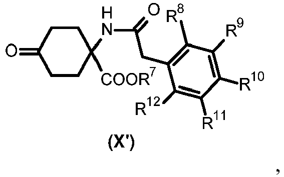 dans laquelle R⁷, R⁸, R⁹, R¹⁰, R¹¹, R¹² présentent les significations indiquées ci-dessus.

5. Composés de formule (XII') 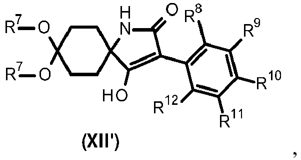 dans laquelle R⁷, R⁸, R⁹, R¹⁰, R¹¹, R¹² présentent les significations indiquées ci-dessus.

6. Composés de formule (VI') 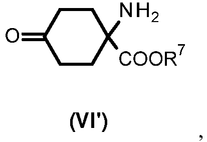 dans laquelle
R⁷ présente la signification indiquée dans la revendication 1.

7. Procédé de préparation de composés de formule (XI), **caractérisé en ce qu'**on transforme des composés de formule (III) par réaction avec des composés de formule (XV)
R⁷-OH (XV),
et du chlorure de thionyle en mélanges des chlorhydrates des composés des formules (IV'), (V') et (VI'), on transforme ces chlorhydrates au moyen de bases en composés libres des formules (IV'), (V') et (VI'), on acyle ensuite ceux-ci en présence d'une base avec des composés de formule (VII), des mélanges des composés des formules (VIII'), (IX') et (X') étant obtenus, on transforme ensuite ces composés en présence d'un acide avec des composés de formule (XIV)
HO- (CR¹R²)ₙ-CR³R⁴-CR⁵R⁶-OH (XIV)
en composés de formule (XVI)
et on cyclise ensuite ces composés dans une réaction de Dieckmann sous l'effet d'une base forte en composés des formules (XI) : 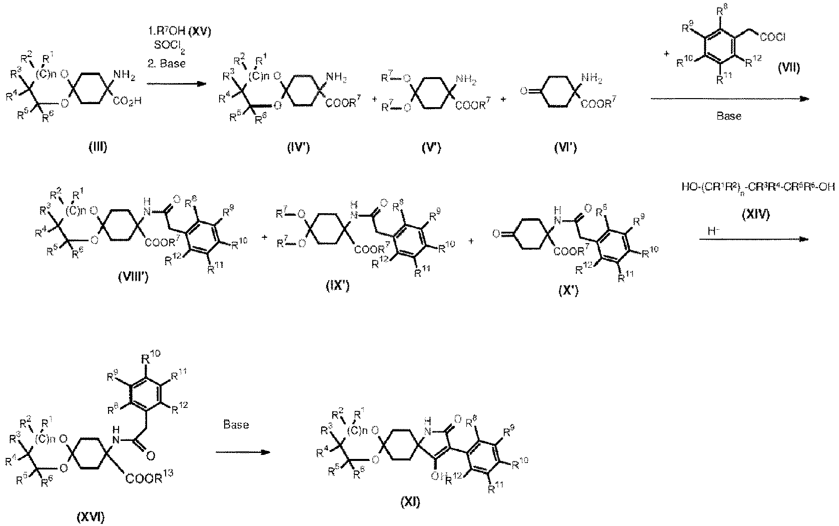 où
R³ représente hydrogène,
R⁴ représente hydrogène,
R⁵ représente hydrogène,
R⁶ représente hydrogène,
R⁷ représente n-propyle,
R⁸ représente méthyle,
R⁹ représente hydrogène,
R¹⁰ représente chlore,
R¹¹ représente hydrogène,
R¹² représente méthyle,
R¹³ représente n-propyle ou -CH₂CH₂-OH,
n vaut 0,
vu que n vaut 0, les substituants R¹ et R² ne sont pas définis.
